# EUROPEAN PATENT SPECIFICATION

(11) **EP 2 971 111 B1**
(45) Date of publication and mention of the grant of the patent: **02.05.2018**
(21) Application number: 14728616.5
(22) Date of filing: 14.03.2014
(51) Int. Cl.: C12Q 1/68

(54) **METHODS FOR THE DETECTION OF BREAKPOINTS IN REARRANGED GENOMIC SEQUENCES**
VERFAHREN ZUM NACHWEIS DER BRUCHSTELLEN IN CHROMOSOMENUMORDNUNGEN
PROCEDE POUR LA DETECTION DES DES POINTS DE RUPTURE DANS DES REARRANGEMENTS CHROMOSOMIQUES

(30) Priority: 15.03.2013 US 201361793944 P
(43) Date of publication of application: 20.01.2016
(73) Proprietor: Genomic Vision, 92220 Bagneux (FR)
(72) Inventor: CEPPI, Maurizio, 81479 München (DE); ABSCHEIDT, Jennifer, 92320 Chatillon (FR); CONSEILLER, Emmanuel, 75015 Paris (FR)
(74) Representative: Desaix, Anne
(86) International application number: PCT/IB2014/000496
(87) International publication number: WO 2014/140789

(56) References cited:
- WO-A1-2013/064895
- FR-A1- 2 755 147
- KEVIN CHEESEMAN ET AL: "A diagnostic genetic test for the physical mapping of germline rearrangements in the susceptibility breast cancer genes BRCA1 and BRCA2", HUMAN MUTATION, vol. 33, no. 6, 4 April 2012 (2012-04-04), pages 998-1009, XP055054668, ISSN: 1059-7794, DOI: 10.1002/humu.22060 & "Cheeseman et al., Human Mutation suppl. data", , 4 April 2012 (2012-04-04), XP055128561, Retrieved from the Internet: URL:http://onlinelibrary.wiley.com/store/1 0.1002/humu.22060/asset/supinfo/humu_22060 _sm_SuppInfo.pdf?v=1&s=c46533e78893a726279 eecbf66dba8048d9daf76 [retrieved on 2014-07-14]
- SYLVIE MAZOYER: "Genomic rearrangements in theBRCA1 andBRCA2 genes", HUMAN MUTATION, vol. 25, no. 5, 1 January 2005 (2005-01-01), pages 415-422, XP055054669, ISSN: 1059-7794, DOI: 10.1002/humu.20169
- GAD SOPHIE ET AL: "Color bar coding the BRCA1 gene on combed DNA: A useful strategy for detecting large gene rearrangements", GENES CHROMOSOMES & CANCER, JOHN WILEY & SONS, INC, US, vol. 31, no. 1, 1 May 2001 (2001-05-01), pages 75-84, XP002512886, ISSN: 1045-2257, DOI: 10.1002/GCC.1120
- SOPHIE GAD ET AL: "Significant contribution of large BRCA1 gene rearrangements in 120 French breast and ovarian cancer families", ONCOGENE, NATURE PUBLISHING GROUP, GB , vol. 21, no. 44 3 October 2002 (2002-10-03), pages 6841-6847, XP002682438, ISSN: 0950-9232, DOI: 10.1038/SJ.ONC.1205685 Retrieved from the Internet: URL:http://www.nature.com/onc/journal/v21/ n44/full/1205685a.html [retrieved on 2014-07-10]
- Genomic Vision extends its patent portfolio with the Genomic Morse Code: "Genomic Vision Press release", , 3 July 2012 (2012-07-03), XP055127903, Retrieved from the Internet: URL:http://www.genomicvision.com/media/CPG enomicMorseCodeENlong.pdf [retrieved on 2014-07-09]

### SEQUENCE LISTING

<110> GENOMIC VISION
<120> METHODS FOR THE DETECTION OF BREAKPOINTS IN REARRANGED GENOMIC SEQUENCES
<130> B10122A AD/PW/BA
<140> PCT/IB/XXXXXX
   <141> 2014-03-14
<150> US 61/793944
   <151> 2013-03-15
<160> 21
<170> PatentIn version 3.5
<210> 1
   <211> 4485
   <212> DNA
   <213> Artificial Sequence
<220>
   <223> Human - Synt1
<400> 1
   ttcagaaaat acatcaccca agttcccatc cctacctgtc tatccacaaa accaaggcat      60
   tcctgagatt agttcattta ttatactaat ataacaagtg tttattaagt atctactact     120
   atattcaagt actattctag gagatagaaa tgtagcagtt tacaaaataa agcctgctct     180
   catagagctc atattctagt gtggtagaca gttgatacgg aattaaagaa tacatgggaa     240
   taagtgcatt aaagagaaaa attaagcagg gtaaggggaa acaggtagtt caatatctat     300
   gtgggggtga gatgtacatg gggggagtca ggaaaggttt cactgaggtg agactagagg     360
   atagcttaat aatgtaaaga aacacactat gcaacaatta ggggaagagc attccaagaa     420
   agagggagca gagaaggcaa accctgagca ggaccatgcc tgtgtatgca ggacatcaga     480
   taggtcaagg tgctaaaatg taataatcca ggaggatatt gtagggaaag actatcagag     540
   aggtagctgg taacttctgg taggaaccta taggctattt taaatcttta gctttattct     600
   ggtcttttta attttctttt tttttttcag acagagtctc gttctgtcgc ccaggctgga     660
   gtgcagtggc accatctcgg ctctctgtaa cctccgcctc ctgaattcaa gtgattctcc     720
   tgcctcagcc tcccgagtag ctgggactaa aggcatgcac caccatgcct tggcctccca     780
   aagtactggg attacaggag tgagccacca tgccagccat ctttttaatt tttaatgtta     840
   attaattttt gtagagacag gatctcacta tgatgcccat gctggtcttg aatgcctggc     900
   atcaagcaat cttcctgctt cggcttccca aagtgctggg attacaggtg tgagctacta     960
   tacccggcct ttagctttct tctgaatgtg aacctttttt tttttttttg gagatggagt    1020
   ctcactcact ctgctgctca ggctggagtg cagtggtgtg gtcttggctc actgcaacct    1080
   ctgcctctcg gattgaagtg attcttgtgc ctcagcattc caagtagctg ggactacagg 1140
   cgcgtgctgc cacacccggc taattttttt gtatttttgg tagggaaggg gtttcaccat 1200
   attgcccagg ctggtcttga agtcctgacc tcaagtgatc catctgcctc gaccgggatt 1260
   acaggcgtga gccactacac ttagctctaa atgtgaattt ttgaaacgga ttttttggat 1320
   aaagtccagg caagatatca aagaacgact aacctggcag tgtgacaaga atgtggtttt 1380
   ttccttaaat atttaacttt ttagaaaagg atcacaaggg ccaggtgcgg tggctcacgc 1440
   tgtaatccca gcattttggg aggccaaggc gggccagcct gggtgacaga gaatccatct 1500
   caaaaaaaga aaaaaaaaaa agaaaaggat cacaagaaaa gcttgtggac agtaacctta 1560
   ttgtgaaggg ttgtaataca actcttgtaa tcatggggtt tttgacatag cacagggcag 1620
   tgaaaagaaa aacaatgaac taagtcagga ggctgggttt ctactaccag ttgtgtatat 1680
   aagcagagcc accttgggct aaccactcta cctgaacctg tttccttctc ttgccattca 1740
   ccctgccaga ctccttgggc tattgcaaga ataaaattaa atgctacttg ggaaaatgct 1800
   tcacaacctg agatgacttg ggaaaaatgc ttcacaacct gagataactt gtaccaacat 1860
   tggtattatt actgggacca aatgtgactt taaaaagaaa aacaaccttg acaaagaaaa 1920
   ctctgattgg ttactaaatc cctatttctg agataagcta catttcaaag aaattctccg 1980
   taaaagaaaa attggattca gttatcatac cagatggctt tcattctcac cactgactca 2040
   attctgaaac aattatattt cagtatggta attataatct aaactatata aacacactgt 2100
   aaacacaaac tttgaacaga tgaaaactcc gatatgtaaa aaggtaatga atgttgaagg 2160
   aagactgtga aaagggaaaa gaaaaaaaat taaaatgttc cccttctagg tcctgatgag 2220
   agtaaatgtt tactataaaa atgattcaaa tattttaaac acttttcaaa ccaggcaata 2280
   ttttaggcct actgtatatt tgcattttga gcttccaata cggataagtg actggaaaaa 2340
   gcagctaggt ttaggttgaa aaacaacaac ccaccgggga acacatttta gcaaattctt 2400
   ctgaaagtca aaaatgttat agtcataggt aaaaagttac aaagaactac caattgtcag 2460
   aaatagctgc caatattgac ttagaagaca gcagaaggaa ttttagttca agaaacctaa 2520
   aacaggctga aaaccttacc taccctatag ctaccacaaa taacactgtt tccagtcatg 2580
   atcattcctg atcacatatt aagacataac tgcaaattgt gctatactgt actatattaa 2640
   aaggaagtga aatatgatcc ctatcctaga actttccata caaatgaatg taaaacacca 2700
   taaaaattaa tcttaaggcc gggcgcggtg gctcacgcct gtaatcccag cactttggga 2760
   ggccgaggtg ggcggatcac gaggtcagga agtggagacc atcctggcta acacggtgaa 2820
   accccgtctc tactaaaaat acaaaaaatt agccgggcgt ggtggtggac gcctgtagtc 2880
   ccagctactt ggggggccga ggcaggagaa tggcgtgaac ccgggaggcg gagcttgcag 2940
   tgagccgaga tggcgccact gcactccggc ctgggtgaaa gagcgagact ccgtctcaaa 3000
   aacaaaacaa acaaaaatta atcttaagcc aggcgcagtg gctcacgcca gcactttgga 3060
   aggccgaggc gggtggatca cgagatcagg acttcaagac cagcctgacc aacgtgatga 3120
   aaccctatct ctactaaaaa tacaaaatta gccggccacg gtggcgtgcg cctataatcc 3180
   cagctactca ggaggctgag gcaggagaag cgcttgaact tgaacctggc aggcggaggt 3240
   tgcagtgagc caagatggcg ccactgcact ccagcctggg cgacagagcc agactccaac 3300
   cccccacccc gaaaaaaaaa ggtccaggcc gggcgcagtg gctcaggact gtaatcccag 3360
   cactttggaa ggctgaggcg ggtggatcac aaggtcagga gatcgagacc atcttggcta 3420
   acatggtgaa accccgtctc tactaaaaat acaaaaaatt agccgggcat agtggtgggc 3480
   gcctgtagtc ccagctactc gggaggctga ggcaggagaa tggcctgaac ccgggaggcg 3540
   gagctggcag tgagccaaga tcgtgccact gcactccagc ctaggcagca gagcgagacc 3600
   gtgtctcaaa aaaacaaaac aaaacaaaac aaaaagtctg ggagcggtgg ctcacgcctg 3660
   taatcccagc actttcggag gccaaggcag gaggatcacc tgaggtcagg agttcgagac 3720
   caacctgacc aatatggaga aaccctgtct ctactaaaaa tacaaaatta gctggtgtga 3780
   tggcacatgc ctgcaatccc aggtactccg gaggctgagg cagcagaatt gcttgaaccc 3840
   gggaggtgga ggttgtagtg agccgagatt gtgccactgc actccagcct gggcaacaag 3900
   agccaaagtc tgtctcaaaa aaaaaaaaaa aaaaaaaaaa agaaattaat cttaacagga 3960
   aacagaaaaa agcaatgaaa agctagaaaa cataatagtt gattgaaaat aacaatttag 4020
   cattttcatt cttacatctt taatttttat gtatctgagt ttttaattga tggtttaatt 4080
   tgccagaatg agaaagaaca tcctattttt atgactctct cccatggaaa tgaaacataa 4140
   atgtatccaa atgccacact attgaggatt ttcctgatca ctgattgtca tgagtaagtt 4200
   ttgtgctttt tcaaaagcag ttttttccta caatgtcatt tcctgcttct ctggctctga 4260
   ttttcaataa attgataaat tgtgaatcct gttttcctct tatttttgtt tagctataat 4320
   gttgaagggc aagggagagg atggttattt ataaatcttg tatcgctctg aaaacacaac 4380
   atacattttc cttaatctga ttaacttgac ttcaaatatg aaaaacaact ttcataaagc 4440
   agaaaagaat ttaccctttt ttattgtggg taagaggcaa tggta 4485
<210> 2
   <211> 3309
   <212> DNA
   <213> Artificial Sequence
<220>
   <223> Human - S7
<400> 2
   agtttagctc tgtcgctgga gttcagtggt gccatattgg ctcacagcaa catctgcctc 60
   ctggttcaag tgattctcct gcctcagcct cctgagtagc tgggattaca ggcacatgcc     120
   actacgccca gctaattttt gtatttttag tggagagggg gtttcaccat gttggccagg     180
   atggtctcga tctcctgacc tcgtgatcct accaccttgg cctcccaaag tgctgggatt     240
   acaggcataa gccaccgccc tcggcctcat ccatgatttt attttgccat ttcaagtgat     300
   ggagcttgtt ttagagctgg aagaaaagcc aaaatgccag ttaatctaaa ctagattcct     360
   gccccagtgc agaaccaatc aagacagagt ccctgtcttt cccggaccac aggatttgtg     420
   ttgaaaagga gaggagtggg agaggcagag tggatggaga acaaggaatc attttctata     480
   tttttaaagt tcttcagtta agaaaatcag caattacaat agcctaatct tactagacat     540
   gtcttttctt ccctagtatg taaggtcaat tctgttcatt tgcataggag ataatcatag     600
   gaatcccaaa ttaatacact cttgtgctga cttaccagat gggacactct aagattttct     660
   gcatagcatt aatgacattt tgtacttctt caacgcgaag agcagataaa tccatttctt     720
   tctgttccaa tgaactttaa cacattagaa aaacatatat atatatcttt ttaaaaggtt     780
   tataaaatga caacttcatt ttatcatttt aaaataaagt aaatttaaga tttggaaggt     840
   tttagaataa tacaaaccaa agaactaatg acaacgtcct ttatttttaa agattctaga     900
   agttgctttt tgtaattaga caacataaat tctgaatttt ttcacatatt gctgccaacc     960
   ccttgggtct tttcctttct ccaagaaaga gaaagctaca gaggagtgac tgaccgggta    1020
   ggtggtggta gccttagctt tctccaatgt ttctggttgt tttctttttc ttgcataaaa    1080
   ccaaaatcaa caacgaccaa accaacacca atcaaggcct ccccgcccct aacctttccc    1140
   agtgacctgc tctcatctct ggatcctcct caagcacatc cctgccggca gcatctgtta    1200
   ctactgacgc tcctctactt ccctcttgcg ctttctcaat ggcgcaaatg gatccagttc    1260
   ttaagttctc cctcccacaa aatcctgtct cctccccttc ccagacatat tcctggcacc    1320
   tcttcttcca caaggtccca tcctctcata cataccagcc ggtgtttttt gttttgtttt    1380
   gttttgtttt gttttgagac agtctcgctc tgtcgcccag gctggagtgc aatggcgcga    1440
   tctcggctca ctgcaacctc cgcctcccgg gttctagcga ttctcctgcc tcagcctcct    1500
   gagtagctgg agcggcacca cgcccggcta atttttgtat ttttagtaga gacggagttt    1560
   caccacgttg gtcaggctgg tctggaactc ctgacctcat gaccagccga cgtttttaaa    1620
   gacatagtgt ccccctcaag gcatattcca gttcctatca cgaggattcc cccacggaca    1680
   ctcagtgccc ccttcctgat cctcagcgct tccctcgcga cctacaaact gcccccctcc    1740
   ccagggttca caacgcctta cgcctctcag gttccgcccc taccccccgt caaagaatac     1800
   ccatctgtca gcttcggaaa tccactctcc cacgccagta ccccagagca tcacttgggc    1860
   cccctgtccc tttcccggga ctctactacc tttacccaga gcagagggtg aaggcctcct    1920
   gagcgcaggg gcccagttat ctgagaaacc ccacagcctg tcccccgtcc aggaagtctc 1980
   agcgagctca cgccgcgcag tcgcagtttt aatttatctg taattcccgc gcttttccgt 2040
   tgccacggaa accaaggggc taccgctaag cagcagcctc tcagaatacg aaatcaaggt 2100
   acaatcagag gatgggaggg acagaaagag ccaagcgtct ctcggggctc tggattggcc 2160
   acccagtctg cccccggatg acgtaaaagg aaagagacgg aagaggaaga attctacctg 2220
   agtttgccat aaagtgcctg ccctctagcc tctactcttc cagttgcggc ttattgcatc 2280
   acagtaattg ctgtacgaag gtcagaatcg ctacctattg tccaaagcag tcgtaagaag 2340
   aggtcccaat cccccactct ttccgcccta atggaggtct ccagtttcgg taaatataag 2400
   taataaggat tgttgggggg gtggagggaa ataattattt ccagcatgcg ttgcggaatg 2460
   aaaggtcttc gccacagtgt tccttagaaa ctgtagtctt atggagagga acatccaata 2520
   ccagagcggg cacaattctc acggaaatcc agtggataga ttggagacct gtgcgcgctt 2580
   gtacttgtca acagttatgg actggagtgt tatgttttcg tattttgaaa gcagaaacta 2640
   ggccttaaaa agatacgtac aactctttag ggagactaca attcccatcc agccccagga 2700
   gtctggggca agtagtcttg taaggtcagt ggcctgcggg gacgcagtga gcgccgaatt 2760
   tgcctggggc aggggaaatg cgctctggcc catgtctgcg cactcgtagt tccacccctc 2820
   agccccagtg tttgttattt ttcgggttca gcttgctttt gccccgtctc cgtcgacgca 2880
   atcgccacca gtcaatgggg tggtcgtttt gagggacaag tggtaagagc caatcttctt 2940
   ggcgaaaacg cggagaaacg ggactagtta ctgtctttgt ccgccatgtt agattcaccc 3000
   cacagagata gcggcagagc tggcagcgga cggtctttgc attgccgcct ccccaggggg 3060
   cgggaagctg gtaaggaagc agcctgggtt agctaggggt ggggtcacgt cacactaaga 3120
   gggtttggag aagttcaagg gaggaatcct gcaaagaaga ggggcgactt tttccgtgtc 3180
   tccggacagc taatcgtttt agtgacagga tgagagagcc cttcgtgttc tgagggaccg 3240
   agtgggcgaa aagcgccgga gagttggaga gtctgtggtt cagaatgcga ggtgacaacg 3300
   tgctagcag 3309
<210> 3
   <211> 20
   <212> DNA
   <213> Artificial Sequence
<220>
   <223> Human - F7
<400> 3
   agggtttcat cacgttggtc 20
<210> 4
   <211> 20
   <212> DNA
   <213> Artificial Sequence
<220>
   <223> Human - R7
<400> 4
   gcaaatgtag tggggacttg 20
<210> 5
   <211> 18
   <212> DNA
   <213> Artificial Sequence
<220>
   <223> Human - F9
<400> 5
   ctgcgcctgg cttaagat 18
<210> 6
   <211> 18
   <212> DNA
   <213> Artificial Sequence
<220>
   <223> Human - R8
<400> 6
   gatgtgggtg gggtcaga 18
<210> 7
   <211> 22
   <212> DNA
   <213> Artificial Sequence
<220>
   <223> Human - F1
<400> 7
   atagggtttc atcacgttgg tc 22
<210> 8
   <211> 20
   <212> DNA
   <213> Artificial Sequence
<220>
   <223> Human - R1
<400> 8
   ctaatctggt gggcacttgg 20
<210> 9
   <211> 22
   <212> DNA
   <213> Artificial Sequence
<220>
   <223> Human - F2
<400> 9
   gtcttgaagt cctgatctcg tg 22
<210> 10
   <211> 20
   <212> DNA
   <213> Artificial Sequence
<220>
   <223> Human - R2
<400> 10
   gtgtctagct tggggtttgg 20
<210> 11
   <211> 22
   <212> DNA
   <213> Artificial Sequence
<220>
   <223> Human - F3
<400> 11
   gagatagggt ttcatcacgt tg 22
<210> 12
   <211> 20
   <212> DNA
   <213> Artificial Sequence
<220>
   <223> Human - R3
<400> 12
   cagatgggga cttggaaaac 20
<210> 13
   <211> 20
   <212> DNA
   <213> Artificial Sequence
<220>
   <223> Human - F4
<400> 13
   gtttcatcac gttggtcagg 20
<210> 14
   <211> 20
   <212> DNA
   <213> Artificial Sequence
<220>
   <223> Human - R4
<400> 14
   ctgagtcaga tggggacttg 20
<210> 15
   <211> 21
   <212> DNA
   <213> Artificial Sequence
<220>
   <223> Human - F5
<400> 15
   gttcaagttc aagcgcttct c 21
<210> 16
   <211> 20
   <212> DNA
   <213> Artificial Sequence
<220>
   <223> Human - F6
<400> 16
   ctgccaggtt caagttcaag 20
<210> 17
   <211> 25
   <212> DNA
   <213> Artificial Sequence
<220>
   <223> Primer Syntl-F
<400> 17
   ttcagaaaat acatcaccca agttc 25
<210> 18
   <211> 22
   <212> DNA
   <213> Artificial Sequence
<220>
   <223> Primer Syntl-R
<400> 18
   taccattgcc tcttacccac aa 22
<210> 19
   <211> 21
   <212> DNA
   <213> Artificial Sequence
<220>
   <223> Primer S7-F
<400> 19
   gagtttagct ctgtcgctgg a 21
<210> 20
   <211> 20
   <212> DNA
   <213> Artificial Sequence
<220>
   <223> Primer S7-R
<400> 20
   tgctagcacg ttgtcacctc 20
<210> 21
   <211> 48
   <212> DNA
   <213> Artificial Sequence
<220>
   <223> Breakpoint 48bp
<400> 21
   gaggcaggag aatggcgtga acccgggagg cggagcttgc agtgagcc 48

## Description

### Field of the Invention

The invention relates to a method for detecting the amplifications of sequences in the BRCA1 locus, which sequences have ends consisting of or are framed with sequence stretches present at least twice in the BRCA1 locus, and which amplification results in at least two or at least three, especially three, tandem copies of the amplified sequence. This invention also relates to methods for determining a predisposition to diseases or disorders associated with these amplifications, including predisposition to ovarian cancer or breast cancer. This invention also relates to a method for detecting amplifications with similar features in other loci.

### Description of the Related Art

Breast cancer is the most common malignancy in women, affecting approximately 10% of the female population. Incidence rates are increasing annually and it is estimated that about 1.4 million women will be diagnosed with breast cancer annually worldwide and about 460,000 will die from the disease. Germline mutations in the hereditary breast and ovarian cancer susceptibility genes BRCA1 (MIM#113705) and BRCA2 (MIM# 600185) are highly penetrant (King et al., 2003), (Nathanson et al., 2001). BRCA1 and BRCA2 genes, together with other genes such as NBR2 gene have been identified, characterized and mapped in the human genome and these data are publicly available. Screening is important for genetic counseling of individuals with a positive family history and for early diagnosis or prevention in mutation carriers. When a BRCA1 or BRCA2 mutation is identified, predictive testing is offered to all family members older than 18 years. If a woman tests negative, her risk becomes again the risk of the general population. If she tests positive, a personalized surveillance protocol is proposed: it includes mammographic screening from an early age, and possibly prophylactic surgery. Chemoprevention of breast cancer with anti-estrogens is also currently tested in clinical trial and may be prescribed in the future.

Most deleterious mutations consist of either small frameshifts (insertions or deletions) or point mutations that give rise to premature stop codons, missense mutations in conserved domains, or splice-site mutations resulting in aberrant transcript processing (Szabo et al., 2000). However, mutations also include more complex rearrangements, including deletions and duplications of large genomic regions that escape detection by traditional PCR-based mutation screening combined with DNA sequencing (Mazoyer, 2005). Only one amplification involving more than two copies has been reported so far (Hogevorst et al., 2003). This amplification is a triplication in the 3' portion of the BRCA1 gene, involving exons 17-19 and caused by Alu recombination.

Techniques capable of detecting these complex rearrangements include Southern blot analysis combined with long-range PCR or the protein truncation test (PTT),quantitative multiplex PCR of short fluorescent fragments (QMPSF) (Hofmann et al., 2002), real-time PCR, fluorescent DNA microarray assays, multiplex ligation-dependent probe amplification (MLPA)(Casilli et al., 2002), (Hofmann et al., 2002) and high-resolution oligonucleotide array comparative genomic hybridization (aCGH) (Rouleau et al., 2007), (Staaf et al., 2008). New approaches that provide both prescreening and quantitative information, such as qPCR-HRM and EMMA, have recently been developed and genomic capture combined with massively parallel sequencing has been proposed for simultaneous detection of small mutations and large rearrangements affecting 21 genes involved in breast and ovarian cancer (Walsh et al., 2010). Other techniques described for the detection of these complex gene rearrangements include Molecular Combing (Herrick and Bensimon, 2009); (Schurra and Bensimon, 2009); (Gad et al., 2001), (Gad et al., 2002a), (Gad et al., 2003); (Cheeseman et al. 2012); (US 61553906).

Prior art methods are unable to detect and/or characterize amplifications when such amplifications involve more than one additional copy of the amplified sequence and/or when the amplified sequence includes portions of sequence present in multiple copies in the wild-type BRCA1 gene or surrounding locus and/or when the amplified sequence belongs to a portion of the BRCA1 locus with very high repeat content. Here, the inventors provide methods to detect and/or characterize such amplifications and to detect and/or characterize amplifications sharing similar features in other genomic loci.

### BRIEF SUMMARY OF THE INVENTION

The BRCA1 and BRCA2 genes are involved, with high penetrance, in breast and ovarian cancer susceptibility. About 2% to 4% of breast cancer patients with a positive family history who are negative for BRCA1 and BRCA2 point mutations can be expected to carry large genomic alterations (in particular deletion or duplication) in one of the two genes, and especially BRCA1. However, some large rearrangements are missed by available techniques. This includes tandem amplification of sequences, characterized by the fact that more than one extra copy of the amplified sequence is introduced and / or characterized by the fact that the extremities of the amplified sequence (the sequence unit which undergoes repetition) are present in multiple copies - either perfectly or strongly homologous to each other - in the wild type locus, and / or when the amplified sequence is in a repeat-rich region.

Methods *in vitro* for detecting and / or characterizing these types of amplifications are one object of the invention . These include *in vitro* methods for detecting the triplication of a sequence fragment encompassing exons 1a, 1b and 2 of BRCA1 and fractions of the NBR2 gene. This region is particularly rich in Alu sequences and common copy number assessing techniques are unable to correctly characterize this triplication. The breakpoints of this tandem triplication share perfect sequence identity over 48 base pairs. This 48 base pair (bp) sequence is found in both BRCA1 and NBR2 genes in the reference human genome sequence. The sequences surrounding this 48-bp sequence show strong homology (80-95 %) over 200-300 bp.

The invention relates to methods for the prediction or for the detection of a breakpoint associated with a rearrangement in a nucleic acid of a biological sample comprising nucleic acid representative of chromosomal nucleic acid, in particular human chromosomal nucleic acid;

The invention relates to tests or methods for this triplication and related amplifications, using Molecular Combing. This direct visualization approach allows immediate detection and characterization of these amplifications, and is not hindered by their repeat sequence content, homologous extremities or the number of copies. The invention also concerns tests or methods, which allow *in vitro* detection and characterization of this triplication and related amplification which are based on enrichment of a biological sample in specific DNA polynucleotides comprising the triplication. These methods are based on polymerase chain reaction (PCR), sequencing and other related techniques. Kits for performing such methods are also within the invention. The methods and kits bring substantial improvement over existing methods which are unable to detect such amplifications.

Results for four unrelated patients are disclosed, showing the triplication in all four patients' samples. The patients were also tested using other techniques of the prior art and the triplication could not be correctly detected or characterized, showing the substantial improvement the inventors brought to existing techniques.

The invention also concerns methods for determining predisposition (also designated as higher risk with respect to a population of reference) to ovarian or breast cancer based on these tests or methods. Furthermore, the inventors describe methods for adapting medical follow-up and / or treatment of patients with increased risk of breast or ovarian cancer and / or patients with ovarian breast cancer linked to this family of amplifications.

Since the 48 bp-sequence constituting the breakpoint for the triplication described herein is also present elsewhere in the BRCA1 gene and surrounding locus, and since sequence amplifications with similar characteristics may be found elsewhere in the genome, the invention concerns methods and kits for detecting such amplifications, bringing substantial improvement over existing methods which are unable to detect such amplifications.

### BRIEF DESCRIPTION OF THE DRAWINGS

The patent or application file contains at least one drawing executed in color.

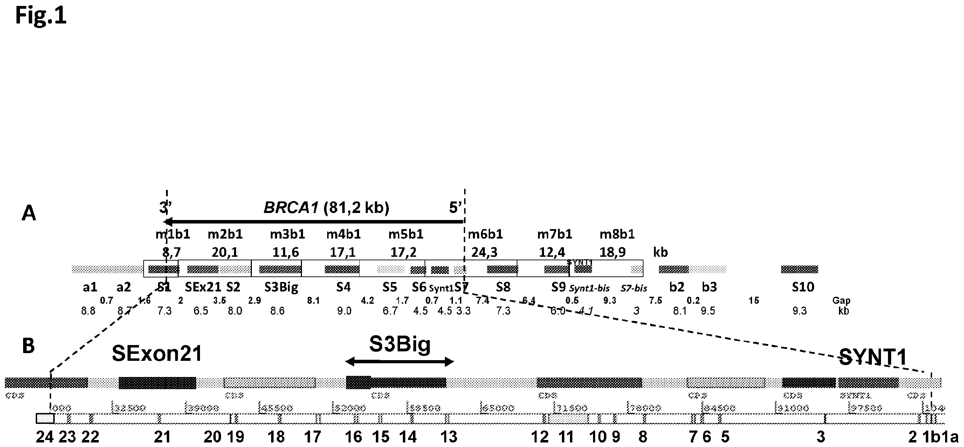

**Figure 1****: *In silico*-generated Genomic Morse Codes 4.0 (GMC 4.0) designed for high-resolution physical mapping of the *BRCA1* genomic region.** (A) The complete *BRCA1* GMC 4.0 covers a genomic region of 200 kb and is composed of 14 signals (a1/a2, S1, Sex21, S2, S3Big, S4, S5, S6, Synt1, S7, S8, S9, b2/b3, S10) of a distinct color (green, red or blue). Each signal is composed of 1 to 2 small horizontal bars, each bar corresponding to a single DNA probe. The region encoding the *BRCA1* (81.2 kb) and *NBR2* (19.5 kb) genes is composed of 8 "motifs" (m1b1-m8b1). Each motif is composed of 1 to 3 small horizontal bars and a black "gap" (no signal). (B) Zoom-in on the *BRCA1* gene-specific signals and relative positions of the 24 exons.

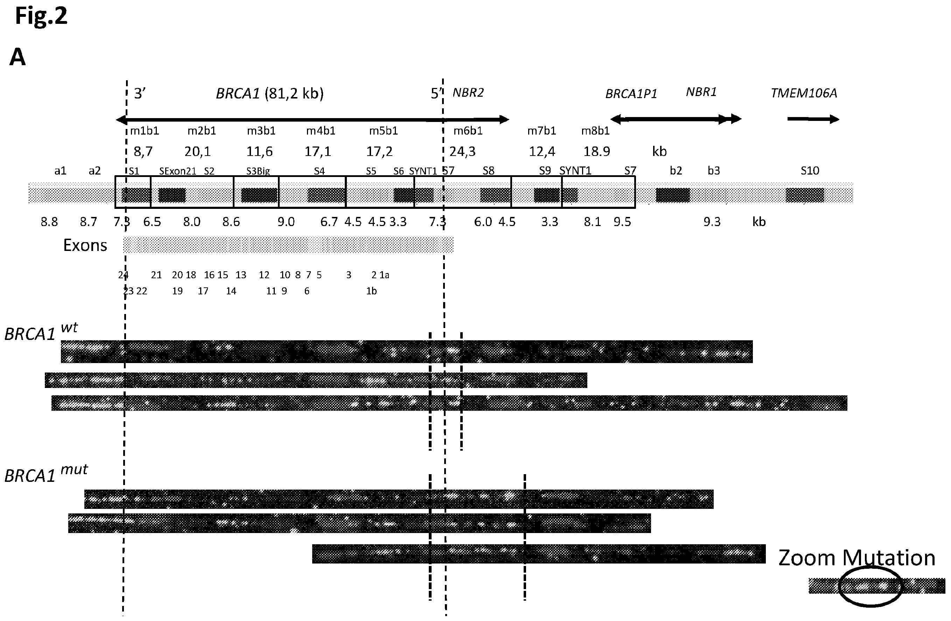

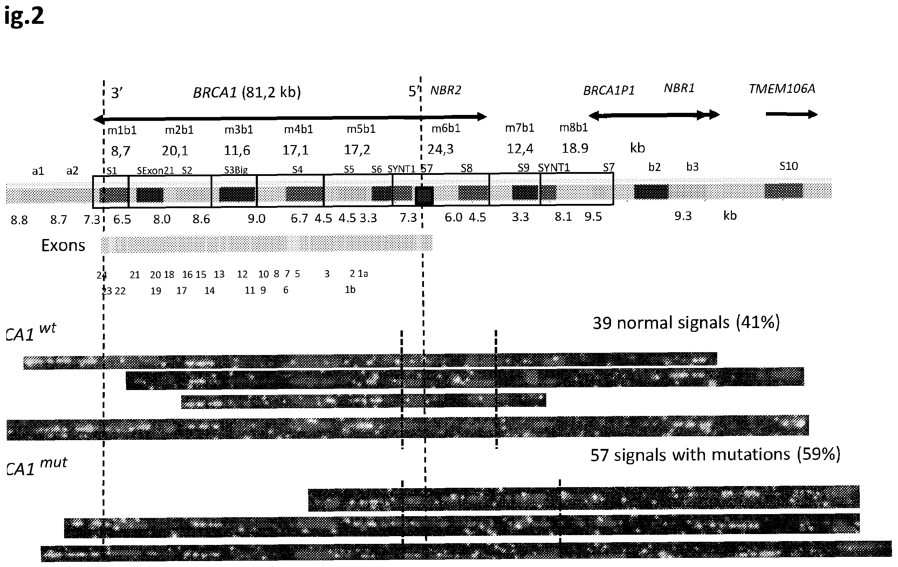

**Figure 2****: Molecular Combing analysis of breast cancer cell-line 10799001.**
   DNA isolated from EBV-immortalized B lymphocytes (cell-line 10799001) collected from a breast cancer patient was analyzed by Molecular Combing.
   (A) BRCA1 v 4.0 GMC computer simulation is shown at the top, the *BRCA1* signals obtained after microscopic visualization are shown at the bottom. 3 microscopy signals are shown for each allele.
      A triplication, visible as a tandem repeat triplication of the red signal SYNT1 and the green signal S7. The position of the detected triplication is indicated with vertical dotted orange lines. *wt* = wild type allele; *mut* = mutated allele bearing triplication.
   (B) Same as (A), but color of DNA probe S7 was switched from green to blue, to confirm the nature of the probe involved in the mutation.

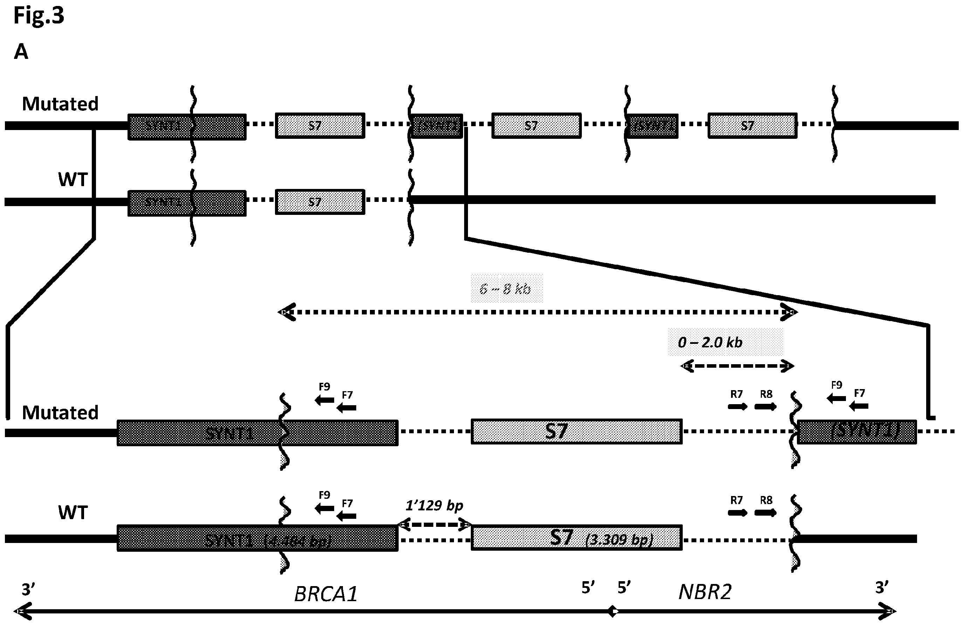

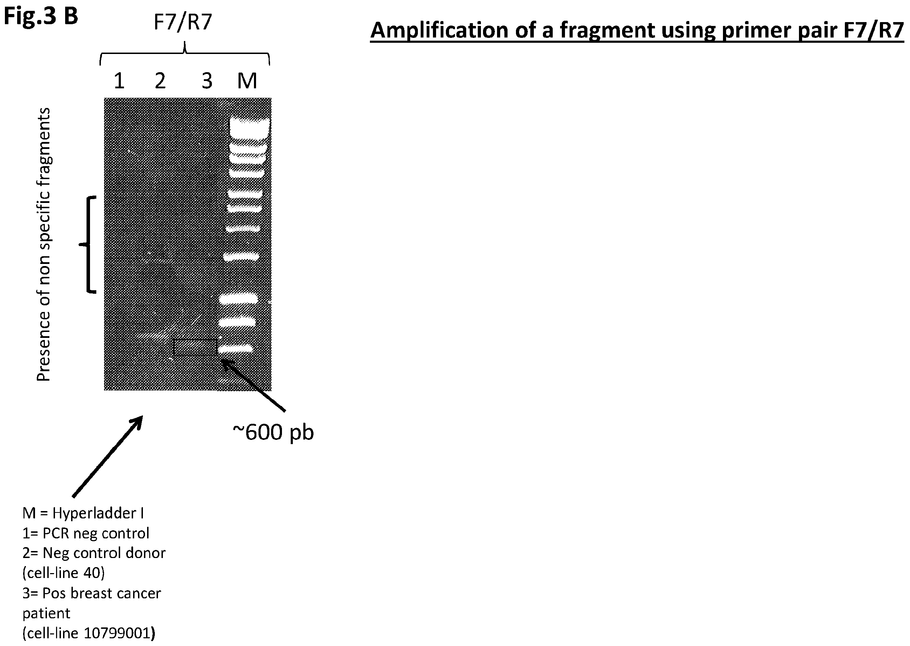

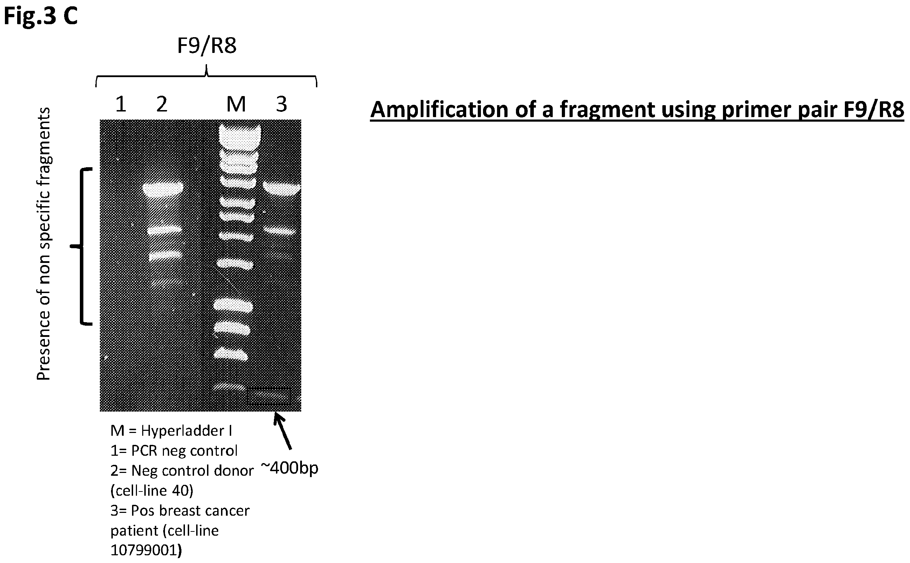

**Figure 3****: Physical mapping of the Triplication of exons 1a, 1b and 2 in *BRCA1.*** (A) Preliminary physical map derived by the Molecular Combing experiments and related measures. Above are the physical maps for the mutated allele (bearing the triplication) and the wild-type allele (corresponding to the reference human genome sequence), with a blown-up view below. The solid line represents the sequence left unchanged in the mutated allele, while the dotted line represents the sequence amplified in the mutated allele. The vertical wavy line is the estimated breakpoint position (and its replicates in the mutated allele). Synt1 and S7 designate full-length signals from the corresponding probes, while *(Synt1)* designates the partial signal arising from the Synt1 probe. Sizes indicatd in bp are the actual size of the probes and gap, while sizes in kb intervals are estimates from Molecular Combing experiments. Four primers are shown as representative examples of primer positioning for the amplification of the breakpoint. (B) - (C) DNA fragments derived after PCR performed in the cell-line 10799001 or the control cell-line 40.

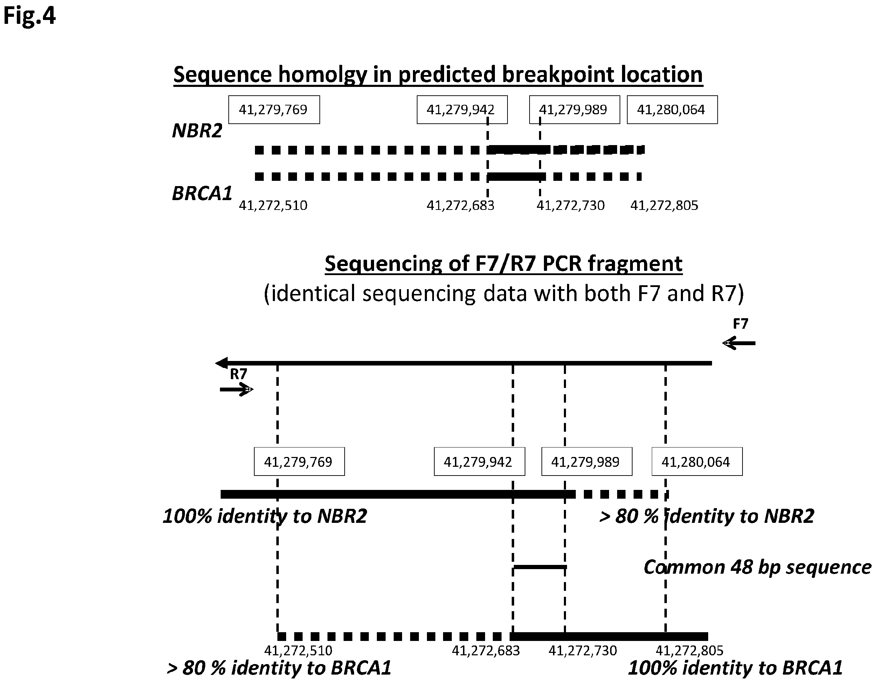

**Figure 4****: Exact physical mapping of the *BRCA1* triplication of exons 1a, 1b and 2.**
   The upper diagram shows the location found to display homology when comparing sequences of the predicted location of both breakpoints, with corresponding genomic coordinates. The overall homology between these 286 bp-sequence stretches is 86.5 %, with a 48-bp portion showing 100 % identity (solid line, and corresponding genomic coordinates).
   The lower diagram shows the results of breakpoint sequencing: sequence identity between sequence data from the F7R7 PCR fragment and the reference human genome sequence is depicted by solid horizontal bars, and sequence homology is depicted by dotted lines, with corresponding genomic coordinates.

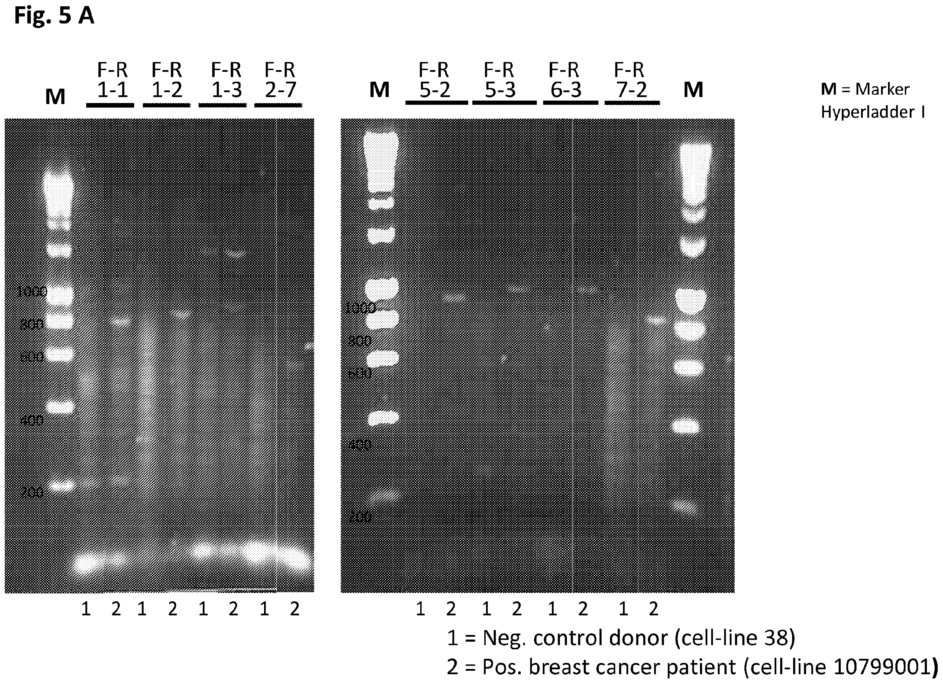

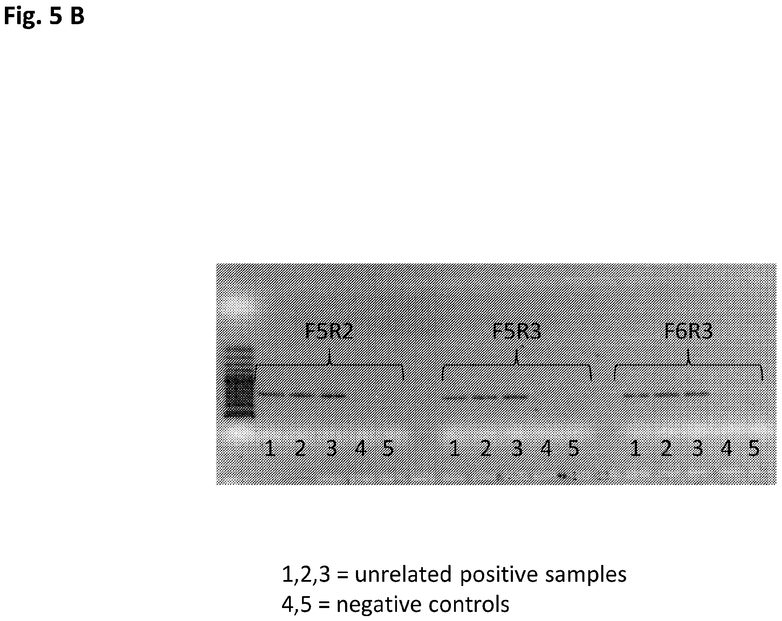

**Figure 5****: Optimized PCR reaction to screen for the *BRCA1* triplication in clinical samples. (A)** Fragments specific for the *BRCA1* triplication were obtained out 8 primers pairs. One single DNA fragment, without any disturbing unspecific fragments, was found for primer pairs F5/R2, F5/R3 and F6/R3 in the mutation positive cell-line 10799001, but not in the control cell-line 38. **(B)** Specific amplification of PCR fragments from primer pairs F5/R2, F5/R3 and F6/R3 observed in 3 unrelated patients harboring the amplification. No PCR product was observed for two negative controls.

### DETAILED DESCRIPTION OF THE INVENTION

The invention relates to methods for the prediction or for the detection of a breakpoint associated with a rearrangement in a nucleic acid of a biological sample comprising nucleic acid representative of chromosomal nucleic acid, in particular human chromosomal nucleic acid;

The invention disclosed herein provides methods for testing *in vitro* the presence of an amplification of a genetic sequence (e.g. stretch of DNA) in a biological sample containing nucleic acid representative of chromosomes, in particular nucleic acid representative of human chromosome 17, and in particular genomic nucleic acid of chromosome 17 comprising:
- submitting said biological sample to a procedure allowing physical mapping of the region extending from exon 2 of the BRCA1 gene to the NBR2 gene;
- detecting more than two successive examples (copies) (duplication or more, in particular triplication) of a 6 kb- to 8 kb-sequence extending from intron 2 of BRCA1 to the NBR2 gene.

The invention also provides kits for testing *in vitro* the presence of an amplification of a genetic sequence in a sample using the method described herein.

The invention relates to a method for *in vitro* prediction of a breakpoint associated with rearrangement, in particular large rearrangement, in a nucleic acid of a biological sample comprising nucleic acid representative of chromosomal nucleic acid, in particular human chromosomal nucleic acid, comprising the steps of:
- mapping the nucleic acid of the biological sample , particularly using Molecular Combing or related direct mapping methods;
- determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
- determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
- within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
- and when such portions exist, exhibiting such sequence identity, reporting that such portions are likely to comprise the breakpoint for sequence rearrangement.

The invention also concerns a method for detection of a breakpoint associated with rearrangement, in particular large rearrangement, in a nucleic acid of a biological sample comprising nucleic acid representative of chromosomal nucleic acid, in particular human chromosomal nucleic acid, comprising the steps of:
- mapping the nucleic acid of the biological sample , particularly using Molecular Combing or related direct mapping methods;
- determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
- determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
- within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
- when such portions exist, exhibiting such sequence identity, concluding that such portions are likely to comprise the breakpoint for sequence rearrangement;
- confirming through molecular testing, in particular through PCR amplification or functionally related method and / or sequencing, the location of the breakpoint.

According to a particular embodiment of the methods according to the invention, the homology and the identity within the nucleic acid of the sample are determined by local alignment search, in particular by successive alignment searches.

In a particular embodiment of the methods according to the invention, the search for homology excludes determining homology for poly-N segments i.e. repeats of a given nucleotide (N), where such a nucleotide is repeated at least 5 times consecutively.

In a particular embodiment the invention relates to a method, wherein the level of homology is within the range of 85 to 95% of identical nucleotides.

In particular, according to method of the invention, the homology is determined on a sequence having 200 to 500bp, in particular 200 to 300bp, in particular about 300 bp.

In a further particular embodiment of the invention, the method as defined herein is such that the prediction or the detection of a breakpoint is associated with a rearrangement consisting of amplification of a nucleic acid sequence, deletion of a sequence in the genomic nucleic acid.

In a particular embodiment of a method of the invention, the prediction or the detection of a breakpoint is performed after detection of a rearrangement in a nucleic acid sequence representative of a human genomic sequence.

In a further particular embodiment of a method according to the invention, the prediction or the detection of a breakpoint is made on a locus of the genome which comprises a gene which is known to be associated with a disease or with a predisposition for a disease, such as genes associated with predisposition to breast and / or ovarian cancer, particularly BRCA1 and BRCA2, genes associated with Lynch syndrome or predisposition to colorectal cancer, particularly MSH2, MLH1, MSH6 and PMS2.

In a specific embodiment of the method of the invention, the breakpoint is detected in the BRCA1 locus.

The invention also concerns a method as defined herein, wherein the confirmation of the breakpoint is performed by PCR using primer pairs selected as follows:
- one forward primer located preferentially less than 5 kb, more preferentially less than 2 kb, even more preferentially less than 1 kb and even more preferentially less than 500 bp from the location of the likely breakpoint at one end of the rearrangement and
- one reverse primer located preferentially less than 5 kb, more preferentially less than 2 kb, even more preferentially less than 1 kb and even more preferentially less than 500 bp from the location of the likely breakpoint at the other end of the rearrangement and where the primers are oriented so that no amplification is possible by PCR in a wild-type sample.

The invention also relates to a method for detecting a predisposition to a disease, or for the detection of a disease, in particular a cancer, especially a breast or ovarian cancer, which comprises performing the prediction or the detection of a breakpoint as defined herein.

The term *"nucleic acid"* and in particular "nucleic acid representative of chromosomes" as used herein designates one or several molecules of any type of nucleic acid capable of being attached to and stretched on a support as defined herein, and more particularly stretched by using molecular combing technology. Nucleic acid, and in particular "nucleic acid representative of chromosomes" also designates one or several molecules of any type of nucleic acid capable of being amplified using PCR or PCR-related methods or capable of being sequenced using sequencing methods. Nucleic acid molecules include DNA (in particular genomic DNA, especially chromosomal DNA, or cDNA) and RNA (in particular mRNA). A nucleic acid molecule can be single-stranded or double-stranded but is preferably double stranded.

*"Nucleic acid representative of a given chromosome"* means that said nucleic acid contains the totality of the genetic information or the essential information with respect to the purpose of the invention, which is present on said chrosomome. In particular, it is chromosomal DNA.

*Physical mapping,* as used herein, is the creation, employing molecular biology techniques, of a genetic map defining the relative position of particular elements such as specified sequence stretches, mutations or markers on genomic DNA. Physical mapping does not require previous sequencing of the analyzed genomic DNA. A physical map obtained by a physical mapping method may include information on the distances or approximate distances separating particular elements or may be limited to information regarding the succession of these elements, i.e. the order in which they appear in the genomic region of interest.

In particular embodiments, the method of the invention involves using FISH or Molecular Combing or related direct mapping methods to allow physical mapping of the region extending from intron 2 of BRCA1 to the NBR2 gene.

*FISH:* Fluorescent *in situ* hybridization.

*Molecular Combing* is a technique for direct visualization of single DNA molecules that are attached, uniformly and irreversibly, to specially treated glass surfaces. Prior to nucleic acid stretching, nucleic acid manipulation generally causes the strand(s) of nucleic acid to break in random locations. Molecular Combing has been described in WO 95/22056, WO 95/21939, WO 2008/028931 and in US 6,303,296.

*Molecular Combing and related direct mapping methods* or *Molecular Combing or related direct mapping methods,* as used herein, designates methods, including Molecular Combing, functionally similar to Molecular Combing, in that they provide means to directly measure distances or approximate distances separating given sequences on single DNA fibers. For some methods, precise determination of the distance between specified sequences is possible. Precise measurement may be understood to provide a distance accurate to 10,000 bp (10 kb), 1,000 bp (1 kb), 100 bp, 10 bp or 1 bp. For other methods, only approximate distance measurements are possible. For other methods yet, only a succession of sequences on a DNA fiber may be determined, i.e. the order in which these sequences are arranged on the DNA fiber, such sequences being possibly present several times on the DNA fiber. While these methods may not always provide means to measure accurately the size of an amplified sequence as addressed herein, they can nevertheless usually detect such amplifications when designed following the method disclosed by the inventors. Molecular Combing and related direct mapping methods may rely on direct measurement of the physical distance between the specified sequences, or on measurement of a physical value directly related to the physical distance between the specified sequences. Such physical values include time, if e.g. the DNA fiber is made to move at a known speed through a detector recording the time of passage of the specified sequences. Such values also include total fluorescence intensity passing through a detector, when such total fluorescence intensity may be related to total nucleic acid content and the DNA fiber is made to move in a detector that can record fluorescence intensity comprised through specified sequences. Such methods may also provide the means for direct reading of the succession of sequences of interest, if e.g. the sequences of interest are labeled with distinct markers or distinct combinations of markers, fluorescent or otherwise, and the method provides means for reading the succession of markers, i.e. the order in which the markers are arranged on the DNA fiber.

In certain embodiments, Molecular Combing and related direct mapping methods are DNA stretching methods. The nucleic acid sample is generally stretched on a support in linear and parallel strands using a controlled stretching factor. By stretching factor it is meant herein the conversion factor allowing to connect physical distances measured on the stretched nucleic acid to the sequence length of said nucleic acid. Such a factor may be expressed as X kb/µm, for example 2kb/µm. By controlled stretching factor it is meant herein a technique for which the stretching factor is sufficiently constant and uniform to allow reliable deduction of the sequence length of a hybridization signal from the measured physical length, with or without the use of calibration probes on the tested sample.

Other DNA stretching methods may be used as an alternative to Molecular Combing. These methods include, for example:
- methods based on the extraction of DNA with detergent and/or high salt concentration, combined or not with the incubation with an intercalating agent and/or UV-light, derived from the methods termed ECF-FISH (extended chromatin fibers-fluorescent in situ hybridization), Halo preparation, and other methods described in (Heng et al., 1992; Haaf and Ward, 1994; Wiegant et al., 1992; Florijn et al., 1995; Vandraager et al., 1998, Raap, 1998, Palotie et al., 1996; Fransz et al., 1996); and
- methods based on the stretching of DNA through the action of a hydrodynamic flow or through mechanical traction on the DNA molecules, by capillarity, gravity or mechanical force, possibly in a micrometer- or nanometerscale device, the DNA being or not immobilized on a solid support, derived from methods termed DIRVISH (direct visual hybridization), optical mapping, and other methods described in Parra and Windle, 1993; Raap, 1998; Heiskanen et al., 1994; Heiskanen et al., 1995; Heiskanen et al., 1996, Mann et al., 1996, Schwartz et al., 1993; Samad et al., 1995, Jing et al., 1998; Dimalanta et al., Palotie et al., 1996; Larson et al., 2006)

In particular embodiments, the method of detection of the invention comprising steps enabling Molecular Combing or related direct mapping method also comprises a hybridization step of nucleic acid representative of chromosome 17, with at least one probe or set of 2 probes or more allowing the identification of the region extending from intron 2 of BRCA1 to the 5' region of NBR2. Hybridization with said probe(s) enables determination of presence of repetition in particular duplication or triplication of amplified sequence of the invention.

In a particular embodiment, the hybridization step is followed by an analysis of the resulting hybridization pattern, consisting of or comprising:
- comparing the resulting hybridization pattern with the theoretical hybridization pattern i.e., the hybridization pattern expected for a wild-type sample;
- in cases where said resulting hybridization pattern contains additional signals when compared to said theoretical hybridization pattern, concluding that the sample contains a sequence amplification;
- optionally, if the probes generating the additional signals cannot be unambiguously identified, performing additional hybridization steps with modified sets of probes allowing the unambiguous identification of the probes generating the additional signals;
- optionally, if said additional signals consist of or comprise several identical patterns, concluding that the sequence amplification resulted in more than one additional copy of the amplified signal.

In particular embodiments, the Molecular Combing or related direct mapping method comprises a hybridization step of nucleic acid representative of chromosome 17, with at least the following probes:
- one probe or set of probes allowing the identification of the intron 2 of the BRCA gene;
- and one probe or set of probes allowing the identification of the 5' region of the NBR2 gene;
- and optionally other probes to confirm the location and / or identify unambiguously the probes or sets of probes above.

As defined herein, a *"probe"* is a polynucleotide, a nucleic acid/polypeptide hybrid, a nucleic acid/polypeptide hybrid or a polypeptide, which has the capacity to hybridize to nucleic acid representative of chromosomes as defined herein, in particular to RNA and DNA by base pairing with said nucleic acid representative of chromosomes which is thus the target for the probe. In a particular embodiment, the probe is substantially or fully complementary to the target nucleic acid and accordingly enables stable hybrids to be formed in stringent conditions of hybridization and detected. This term encompasses RNA (in particular mRNA) and DNA (in particular cDNA or genomic DNA) molecules as well as , peptide nuclear acid (PNA), and protein domains. Said polynucleotide or nucleic acid hybrid generally comprises or consists of at least 100, 300, 500 nucleotides, preferably at least 700, 800 or 900 nucleotides, and more preferably at least 1, 2, 3, 4 or 5 kb. For example probes of 1, 2, 3, 4, 5, 6, 7, 8, 9, 10, 11, 12, 13, 14, 15 kb or more than 15kb, in particular 30, 50 or 100 kb can be used. In a particular embodiment, the length of the probes used is ranging from 0.5 to 50 kb, preferably from 1 to 30 kb and more preferably from 1 to 10 kb, from 4 to 20 kb, from 4 to 10 kb, or from 5 to 10 kb. Said polypeptide generally specifically binds to a sequence of at least 6 nucleotides, and more preferably at least 10, 15, 20 nucleotides. As used herein, the sequence of a probe, when the probe is a polypeptide, should be understood as the sequence to which said polypeptide specifically binds. A probe specific for a given region of the genome or specific for a given sequence, as used herein, is a probe capable in certain conditions of hybridizing on said given region of the genome or on said given sequence while in the same conditions it does not hybridize to most other regions of the genome or to sequences significantly different from said sequence.

In a particular embodiment, the sequence of a probe is at least 99% complementary, i.e., at least 99% identical, or at least 99% similar to the sequence of a portion of one strand of the target nucleic acid to which it must hybridize.

The term *"complementary sequences"* in the context of the invention means "complementary" and "reverse" or "inverse" sequences, i.e. the sequence of a DNA strand that would bind by Watson-Crick interaction to a DNA strand with the said sequence.

Generally, a probe will be tagged or labeled with a marker, such as a chemical or radioactive market that permits it to be detected once bound to its complement. The probes described herein are generally tagged with a visual marker, such as a fluorescent dye having a particular color such as blue, green or red dyes. Some probes according to the invention are selected to recognize particular portions or segments of the BRCA1 gene and surrounding locus.

In a particular embodiment, the nucleic acid sample used for Molecular Combing or related direct mapping methods is genomic DNA, in particular total genomic DNA or more preferably chromosomal genomic DNA (nuclear genomic DNA), and/or fragments thereof. Said fragments can be of any size, the longest molecules reaching several megabases (thousands of kb). Said fragment are generally comprised between 10 and 2000 kb, more preferably between 200 and 700 kb and are in average of about 300 kb.

The nucleic acid sample used in the method of the invention can be obtained from a biological fluid or from a tissue of biological origin, said biological sample, including tissue, being isolated for example from a human (also called patient herein).

*Sequence lengths* are expressed herein in *kb* (kilo base pairs, i.e. 1000 base pairs) or *bp* (base pairs). The length of genetic sequences is usually measured on double stranded nucleic acid and thus expressed in base pairs, where every base pair is made of one nucleotide on one strand and its complementary nucleotide on the other strand. If applied to a single-stranded nucleic acid, the measurement in base pairs is understood to correspond to the measurement of the corresponding double-stranded nucleic acid, i.e. the nucleic acid made of the single-stranded nucleic acid of interest paired with its reverse complementary nucleic acid.

In a particular embodiment, the invention consists of or comprises:
- hybridizing a nucleic acid representative of chromosome 17 with a set of probes including at least one probe or set of probes allowing to identify the region extending from intron 2 of BRCA1 to the NBR2 gene or a portion of this region;
- measuring the size of the region recognized by said probe or set of probes;
- comparing the measured size with the size of a single copy of said region or said portion of said region,
- in the case where the measured size is greater than the size of a single copy of said region or said portion of said region, concluding that the sample contains a sequence amplification in said region;
- and, optionally, if the measured size is greater than the expected size of two tandem copies of said region or said portion of said region, concluding that the sample contains a sequence amplification in said region, with more than one additional copy of the amplified sequence.

In a particular embodiment, the hybridization step is followed by an analysis step consisting of or comprising:
- determining the location of the breakpoint on one end of the amplified sequence and/or a confidence interval for the location of said breakpoint;
- determining the size of the amplified sequence and / or a confidence interval for the size of the amplified sequence;
- determining from the above location and size and / or confidence intervals for the location and / or size the location and / or a confidence interval for the location of the breakpoint at the other end of the amplified sequence.

The invention disclosed herein also provides methods for testing *in vitro* the presence of an amplification of a genetic sequence in a patient's genome, such method comprising:
- obtaining a DNA sample from the patient;
- submitting the DNA sample to a procedure allowing physical mapping of the genomic region extending from intron 2 of the BRCA1 gene to the NBR2 gene;
- detecting more than two successive copies of a 6 kb- to 8 kb-sequence extending from intron 2 of BRCA1 to the NBR2 gene.

The invention also provides kits for testing *in vitro* the presence of an amplification of a genetic sequence in a patient's genome using the method described in the previous paragraph.

*Wild-type:* this expression designates an unmodified sequence for a given gene or genomic region, i.e. the gene or genomic region bearing the sequence published in the reference human genome sequence. Since only large rearrangements are considered herein, where more than 1 kb of sequence have been modified (deleted, amplified, inverted or modified otherwise) relative to the reference sequence, the expression wild-type designates a sequence with less than 1 kb differing from the reference human genome sequence.

*PCR:* polymerase chain reaction

*PCR and related methods:* as used herein, this expression designates any method allowing the detection in a sample and optionally the quantification of one or several fragments of DNA characterized by the sequences of their extremities and itheir sizes. This includes but is not restricted to PCR, quantitative PCR, isothermal amplification (Gill and, Ghaemi, 2008), multiplex, ligation-dependent probe amplification (MLPA, . Schouten et al., 2002)

*Breakpoint:* as used herein, this expression designates the position in the genome of the extremities of a rearrangement found in a DNA sample. This implies that on one side of a breakpoint, the sequence of the DNA sample is identical to the reference human genome sequence, while on the other side the sequence differs from the wild-type sequence. A sequence overlapping the breakpoint would also differ from the reference human genome sequence.

*Reference human genome sequence:* the reference sequence used herein is the human genome Build GRCh37/hg19, available at http://genome.ucsc.edu, on March 1^{st}, 2013.

*genomic position:* genomic positions are given as nucleotide positions corresponding to the reference human genome numbering. *Genomic coordinates* is used herein with the same meaning. Unless otherwise specified, genomic coordinates or positions given herein are from chromosome 17. A genomic position is described herein as "upstream" of another position on the same arm of a chromosome if it is located closer to the centromere (e.g. has a smaller position number if both are on the "q" arm of chromosome 17). Conversely, a genomic position is described as "downstream" of another position on the same arm of a chromosome if it is located further from the centromere (e.g. has a larger position number if both are on the "q" arm of chromosome 17).

*Adaptation of medical follow-up:* as used herein, this expression designates the modification of medical or clinical surveillance for a patient when e.g. the risk of cancer in this patient or predisposition is increased relatively to the general population. For example, a periodic monitoring of biological or clinical characteristics may be advisable for the general population with a given frequency (e.g. in the case of breast cancer, mammographies may be recommended every 5 years), while this monitoring may be advisable with higher frequency for patients at elevated risk of a disease (e.g. in the case of an elevated risk or breast cancer, mammographies may be recommended every year). The adaptation of medical follow-up may be the prescription or recommendation of an adapted follow-up - whether the patient follows the prescription or recommendation or not -; the implementation of the adapted follow-up, or any other action performed aiming to adapt medical follow-up.

*Predictive genetic testing:* screening procedure involving direct analysis of DNA molecules isolated from human biological samples (e.g.: blood), used to detect gene mutations associated with disorders that appear after birth, often later in life. These tests can be helpful to people who have a family member with a genetic disorder, but who have no features of the disorder themselves at the time of testing. Predictive testing can identify mutations that increase a person's chances of developing disorders with a genetic basis, such as certain types of cancer.

*Polynucleotides:* This term encompasses naturally occurring DNA and RNA polynucleotide molecules (also designated as sequences) as well as DNA or RNA analogs with modified structure, for example, that increases their stability. Genomic DNA used for Molecular Combing will generally be in an unmodified form as isolated from a biological sample. Polynucleotides, generally DNA, used as primers may be unmodified or modified, but will be in a form suitable for use in amplifying DNA. Similarly, polynucleotides used as probes may be unmodified or modified polynucleotides capable of binding to a complementary target sequence. This term encompasses polynucleotides that are fragments of other polynucleotides such as fragments having 5, 10, 15, 20, 30, 40, 50, 75, 100, 200 or more contiguous nucleotides.

*BRCA1 locus:* This locus encompasses the coding portion of the human BRCA1 gene (gene ID: 672, Reference Sequence NM_007294) located on the long (q) arm of chromosome 17 at band 21, from base pair 41,196,311 to base pair 41,277,499, with a size of 81 kb (reference genome Build GRCh37/hg19), as well as its introns and flanking sequences. Following flanking sequences have been included in the BRCA1 GMC: the 102 kb upstream of the BRCA1 gene (from 41,277,500 to 41,379,500) and the 24 kb downstream of the BRCA1 gene (from 41,196,310 to 41,172,310). Thus the BRCA1 GMC covers a genomic region of 207 kb.

*BRCA1 gene and surrounding locus: this expression designates herein the human genome portion containing the BRCA1 gene and ∼300 kb flanking portions on either side and corresponds to genomic positions 40,900, 000 to 41,600,000.*

*Intron 2 of BRCA1:* as used herein,this expression designates the genome region comprised between exon 2 and exon 3 of BRCA1, or between genomic positions 41,267,770 and 41,276,000.

NBR2 gene: this gene is mapped in the human genome reference sequence to positions 41,277,600-41,292,342. As used herein, the 5' region of NBR2 is the genomic region comprised between positions 41,277,600 and 41,282,600

*A sequence extending from intron2 of BRCA1 to the NBR2 gene:* this expression designates a sequence having one extremity in the intron 2 of BRCA1 and one extremity in the NBR2 gene. Such a sequence would necessarily include exons 1a, 1b and 2 of BRCA1. Such a sequence would have one extremity located upstream of genomic position 41,276,000 and one extremity located downstream of genomic position 41,277,600.

*Region extending from intron2 of BRCA1 to the NBR2 gene: this expression designates the human genome portion extending from genomic positions 41,270,000 (a position located between exons 2 and 3 of BRCA1) to 41,282,600 (a position located in the NBR2 gene).*

*Germline rearrangements:* genetic mutations involving gene rearrangements occurring in any biological cells that give rise to the gametes of an organism that reproduces sexually, to be distinguished from somatic rearrangements occurring in somatic cells..

*Amplified sequence* encompasses within the invention a stretch of DNA which undergoes repetition (i.e. is copied) in a genome and in particular is repeated so that at least two identical stretches of said DNA, or at least three identical stretches of DNA are present in the considered genome or genomic locus. In particular, the considered stretch of DNA is duplicated (1 additional copy of the stretch of DNA are present, i.e., a same sequence is present two times in the genome or genomic locus) or triplicated (2 additional copies of the stretch of DNA are present, i.e. a same sequence is present three times in the genome or genomic *locus)..Tandem amplification:* mutations characterized by a stretch of DNA that is duplicated to produce two or more adjacent copies, resulting in a tandem repeat array.

*Tandem repeat array:* a stretch of DNA consisting of two or more adjacent copies of a sequence. A single copy of this sequence in the repeat array is called a *repeat unit.* Gene amplifications occurring naturally are usually not completely conservative, i.e. in particular the extremities of the repeated units may be rearranged, mutated and / or truncated. In the present invention, two or more adjacent sequences with more than 90 % homology are considered a repeat array consisting of equivalent repeat unit. Unless otherwise specified, no assumptions are made on the orientation of the repeat units within a tandem repeat array. Such repeat units within a tandem repeat array may be separated by less than 100, or less than 10, or less than 5 or 0 nucleotides that do not belong to the repeated sequence.

*Complex Rearrangements:* any gene rearrangement that can be distinguished from a simple deletion or a simple duplication. Examples are translocations or inversions, or combinations of several duplications, or combinations of deletions and duplications.

*Detectable label or marker*: any molecule that can be attached to a polynucleotide and which position can be determined by means such as fluorescent microscopy, enzyme detection, radioactivity, etc, or described in the US application nr. US2010/0041036A1 published on 18 February 2010.

*Primer:* This term has its conventional meaning as a nucleic acid molecule (also designated sequence) that serves as a starting point for polynucleotide synthesis. In particular, Primers may have 20 to 40 nucleotides in length and may comprise nucleotides which do not base pair with the target, providing sufficient nucleotides in their 3'-end, especially at least 20, hybridize with said target. The primers of the invention which are described herein are used in pairs in PCR procedures, or individually for sequencing procedures.

*Genomic Morse Code(s)*: A GMC is a series of "dots" (DNA probes with specific sizes and colors) and "dashes" (uncolored spaces with specific sizes located between the DNA probes), designed to physically map a particular genomic region. The GMC of a specific gene or locus is characterized by a unique colored "signature" that can be distinguished from the signals derived by the GMCs of other genes or loci. The design of DNA probes for high resolution GMC requires specific bioinformatics analysis and the physical cloning of the genomic regions of interest in plasmid vectors. Low resolution CBC has been established without any bioinformatics analysis or cloning procedure.
*Repetitive sequences:* the *BRCA1* and *BRCA2* gene loci contain repetitive sequences of different types: SINE, LINE, LTR and Alu. Such repetitive sequences are known to make molecular testing difficult due e.g. to non-specific binding of primers. Such repetitive sequences, and regions rich in repetitive sequences, are known to be prone to rearrangements, potentially due to homologuous recombination or similar mechanisms (van Binsbergen et al. 2011).

The term *"sample"* or "biological sample" as used herein relates to a material or mixture of materials, typically, although not necessarily, in fluid form, containing one or more components of interest. For Molecular Combing, the sample will contain genomic DNA from a biological source, in particular suitable for for diagnostic applications, usually obtained from a patient. The invention concerns means, especially polynucleotides, and methods suitable for *in vitro* implementation on samples.

The terms *"nucleoside"* and *"nucleotide"* are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, *e.g.,* wherein one or more of the hydroxyl groups are replaced with halogen atoms or aliphatic groups, or are functionalized as ethers, amines, or the like.

The term *"stringent conditions"* as used herein refers to conditions that are compatible to produce binding pairs of nucleic acids, *e.g.,* surface bound and solution phase nucleic acids, of sufficient complementarity to provide for the desired level of specificity in the assay while being less compatible to the formation of binding pairs between binding members of insufficient complementarity to provide for the desired specificity. Stringent assay conditions are the summation or combination (totality) of both hybridization and wash conditions.

A *"stringent hybridization"* and "stringent hybridization wash conditions" in the context of nucleic acid hybridization (*e.g.,* as required for Molecular Combing or for identifying probes useful for GMC) are sequence dependent, and are different under different experimental parameters. Stringent hybridization conditions that can be used to identify nucleic acids within the scope of the invention can include for example hybridization in a buffer comprising 50% formamide, 5X SSC, and 1% SDS at 42°C, or hybridization in a buffer comprising 5.times.SSC and 1% SDS at 65°C., both with a wash of 0.2X SSC and 0.1% SDS at 65°C. Exemplary stringent hybridization conditions can also include a hybridization in a buffer of 40% formamide, 1M NaCl, and 1% SDS at 37°C, and a wash in 1X SSC at 45°C. Alternatively, hybridization to filter-bound DNA in 0.5 MNaHP0₄, 7% sodium dodecyl sulfate (SDS), 1 mM EDTA at 65°C, and washing in 0.1X SSC/0.1% SDS at 68°C can be employed. Yet additional stringent hybridization conditions include hybridization at 60°C. or higher and 3X SSC (450 mM sodium chloride/45 mM sodium citrate) or incubation at 42°C. in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

A probe or primer *located* in a given genomic locus means a probe or a primer which hybridizes to the sequence in this locus of the human genome. Generally, probes are double stranded and thus contain a strand that is identical to and another that is reverse complementary to the sequence of the given locus. A primer is single stranded and unless otherwise specified or indicated by the context, its sequence is identical to that of the given locus. When specified, the sequence may be reverse complementary to that of the given locus. In certain embodiments, the stringency of the wash conditions that set forth the conditions that determine whether a nucleic acid is specifically hybridized to a surface bound nucleic acid. Wash conditions used to identify nucleic acids may include for example a salt concentration of about 0.02 molar at pH 7 and a temperature of at least about 50°C or about 55°C to about 60°C; or a salt concentration of about 0.15 M NaCl at 72°C. for about 15 minutes; or a salt concentration of about 0.2X SSC at a temperature of at least about 50°C. or about 55°C. to about 60°C. for about 15 to about 20 minutes; or, the hybridization complex is washed twice with a solution with a salt concentration of about 2X SSC containing 0.1% SDS at room temperature for 15 minutes and then washed twice by 0.1X SSC containing 0.1% SDS at 68°C. for 15 minutes; or, equivalent conditions. Stringent conditions for washing can also be for example 0.2X SSC/0.1% SDS at 42°C. A specific example of stringent assay conditions is rotating hybridization at 65°C in a salt based hybridization buffer with a total monovalent cation concentration of 1.5 M followed by washes of 0.5X SSC and 0.1X SSC at room temperature. Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may be employed, as appropriate.

*"Sensitivity"* describes the ability of an assay to detect the nucleic acid of interest in a sample. For example, an assay has high sensitivity if it can detect a small concentration of the nucleic acid of interest in sample. Conversely, a given assay has low sensitivity if it only detects a large concentration of the nucleic acid of interest in sample. A given assay's sensitivity is dependent on a number of parameters, including specificity of the reagents employed (such as types of labels, types of binding molecules, etc.), assay conditions employed, detection protocols employed, and the like. In the context of Molecular Combing and GMC hybridization, sensitivity of a given assay may be dependent upon one or more of: the nature of the surface immobilized nucleic acids, the nature of the hybridization and wash conditions, the nature of the labeling system, the nature of the detection system, etc.

The invention thus relates to each and any of the following embodiments taken individually or in any combination. In particular, the invention concerns the following methods.

Optionnaly, the method of the invention comprises specifying breakpoint location by statistical calculations.
Optionnaly, the method of the invention comprises specifying breakpoint by sequence comparison of regions suspected to contain the breakpoint.
Optionnaly, the method of the invention comprises identifying potential breakpoints as sequences with >80 % homology, over > 200 bp, comprising a stretch of > 25 bp with 100 % identity.
Optionnaly, the method of the invention comprises further specifying / confirming breakpoint location by PCR and related methods and/or sequencing.

### EXAMPLES

### 1. Materials and methods

### Preliminary patient screening

Total human genomic DNA was obtained from the EBV-immortalized lymphoblastoid cell lines nr. 10799001, 38 and 40 obtained from the Institut Curie (Paris). Preliminary screening for large rearrangements was performed with the QMPSF assay (Quantitative Multiplex PCR of Short Fluorescent Fragments) in the conditions described by Casilli et al and Tournier et al (Casilli et al., 2002) and by MLPA (Multiplex Ligation-Dependent Probe Amplification) using the SALSA MLPA kits P002 (MRC Holland, Amsterdam, The Netherlands) for BRCA1 and P045 (MRC-Holland) for BRCA2. The patient gave his written consent for BRCA1 analysis.

### Molecular Combing

### Sample preparation

Total human genomic DNA was obtained from EBV-immortalized lymphoblastoid cell lines. A 45-µL suspension of 106 cells in PBS was mixed with an equal volume of 1.2% Nusieve GTG agarose (Lonza, Basel, Switzerland) prepared in 1x PBS, previously equilibrated at 50°C. The plugs were left to solidify for 30 min at 4°C, then cell membranes are solubilised and proteins digested by an overnight incubation at 50°C in 250 µL of 0.5 M EDTA pH 8.0, 1% Sarkosyl (Sigma-Aldrich, Saint Louis, MO, USA) and 2 mg/mL proteinase K (Eurobio, Les Ulis, France), and the plugs were washed three times at room temperature in 10 mM Tris, 1 mM EDTA pH 8.0. The plugs were then either stored at 4°C in 0.5 M EDTA pH 8.0 or used immediately. Stored plugs were washed three times for 30 minutes in 10 mM Tris, 1 mM EDTA pH 8.0 prior to use.

### Probe preparation

All BRCA1 probes were cloned into pCR2.1-Topo or pCR-XL-Topo (Invitrogen) plasmids by TOPO cloning, using PCR amplicons as inserts. Amplicons were obtained using bacterial artificial chromosomes (BACs) as template DNA. For BRCA, the 207-kb BAC RP11-831F13 (ch17: 41172482-41379594, InVitrogen, USA) was used for probe cloning. Whole plasmids were used as templates for probe labelling by random priming. Briefly, for biotin (Biot) labeling, 200 ng of template was labelled with the DNA Bioprime kit (Invitrogen) following the manufacturer's instructions, in an overnight labelling reaction. For Alexa-488 (A488) or digoxigenin (Dig) labeling, the same kit and protocol were used, but the dNTP mixture was modified to include the relevant labeled dNTP, namely Dig-11-dUTP (Roche Diagnostics, Meylan, France) or A488-7- OBEA-dCTP (Invitrogen) and its unlabelled equivalent, both at 100 µM, and all other dNTPs at 200 µM. Labelled probes were stored at 20°C. For each coverslip, 5 µL of each labelled probe (1/10th of a labelling reaction product) was mixed with 10 µg of human Cot 1 and 10 µg of herring sperm DNA (both from Invitrogen) and precipitated in ethanol. The pellet was then resuspended in 22 µL of 50% formamide, 30% Blocking Aid (Invitrogen), 1X SSC, 2.5% Sarkosyl, 0.25% SDS, and 5 mM NaCl.

*Synt1*: the Synt1 probe described herein is the result of a PCR amplification using BAC RP11-831F13 as a template and the two following primers: Synt1-F (TTCAGAAAATACATCACCCAAGTTC) (SEQ ID NO:**17**) and Synt1-R (TACCATTGCCTCTTACCCACAA) (SEQ ID NO: **18**). The predicted sequence of the Synt1 probe is as follows (corresponding to genomic coordinates 41,269,785-41,274,269):

S7: the S7 probe described herein is the result of a PCR amplification using BAC RP11-831F13 as a template and primers corresponding to the reference human genome sequence at positions 41,275,399 (forward primer : GAGTTTAGCTCTGTCGCTGGA) (SEQ ID NO:**19**) and 41,278,707 (reverse primer: TGCTAGCACGTTGTCACCTC) (SEQ ID NO:20). The predicted sequence of the S7 probe is as follows (corresponding to genomic coordinates 41275399- 41278707):

### Genomic DNA combing and probe hybridisation

Genomic DNA was stained by 1h incubation in 40 mM Tris, 2 mM EDTA containing 3 µM Yoyo-1 (Invitrogen, Carlsbad, CA, USA) in the dark at room temperature. The plug was then transferred to 1 mL of 0.5 M MES pH 5.5, incubated at 68°C for 20 min to melt the agarose, and then incubated at 42°C overnight with 1.5 U beta agarase I (New England Biolabs, Ipswich, MA, USA). The solution was transferred to a combing vessel already containing 1M1 of 0.5 M MES pH 5.5, and DNA combing was performed with the Molecular Combing System on dedicated coverslips (Combicoverslips) (both from Genomic Vision, Paris, France). Combicoverslips with combed DNA are then baked for 4 h at 60°C. The coverslips were either stored at -20°C or used immediately for hybridisation. The quality of combing (linearity and density of DNA molecules) was estimated under an epi-fluorescence microscope equipped with an FITC filter set and a 40 x air objective. A freshly combed coverslip is mounted in 20µL of a 1ml ProLong-gold solution containing 1µL of Yoyo-1 solution (both from Invitrogen). Prior to hybridisation, the coverslips were dehydrated by successive 3 minutes incubations in 70%, 90% and 100% ethanol baths and then air-dried for 10 min at room temperature. The probe mix (20 µL; see Probe Preparation) was spread on the coverslip, and then left to denature for 5 min at 90°C and to hybridise overnight at 37°C in a hybridizer (Dako). The coverslip was washed three times for 5 min in 50% formamide, 1X SSC, then 3 x 3 min in 2X SSC. Detection was performed with two or three successive layers of flurorophore or streptavidin -conjugated antibodies, depending on the modified nucleotide employed in the random priming reaction (see above). For the detection of biotin labelled probes the antibodies used were Streptavidin-A594 (InVitrogen, Molecular Probes) for the 1st and 3rd layer, biotinylated goat anti-Streptavidin (Vector Laboratories) for the 2nd layer; For the detection of A488-labelled probes the antibodies used were rabbit anti-A488 (InVitrogen, Molecular Probes) for the 1st and goat anti-rabbit A488 (InVitrogen, Molecular Probes) for the 2nd layer; For the detection of digoxygenin labelled probes the antibodies used were mouse anti-Dig (Jackson Immunoresearch) for the 1st layer, rat anti-mouse AMCA (Jackson Immunoresearch) for the 2nd layer and goat anti-mouse A350 (InVitrogen, Molecular Probes) for the 3rd Layer. We performed a 20 minutes incubation step at 37°C in a humid chamber for each layer, and three successive 3 minutes washes in 2X SSC, 0.1% Tween at room temperature between layers. Three additional 3 minutes washes in PBS and dehydration by successive 3 minutes washes in 70%, 90% and 100% ethanol were performed before mounting the coverslip.

### Image acquisition

Image acquisition was performed with a customized automated fluorescence microscope (Image Xpress Micro, Molecular Devices, Sunnyvale, CA, USA) at 40x magnification, and image analysis and signal measurement were performed with the softwares ImageJ (http://rsbweb.nih.gov/ij) and JMeasure (Genomic Vision, Paris, France). Hybridisation signals corresponding to the BRCA1 probes were selected by an operator on the basis of specific patterns made by the succession of probes. For all motifs signals belonging to the same DNA fiber, the operator identified the ends of each segment and determined its identity and length (kb), on a 1:1 scale image. The data were then output in a spreadsheet. In the final analysis, only intact signals were considered, i.e. signals where no fiber breakage had occurred within the BRCA1 motifs.

### Statistical analysis

Molecular Combing allows DNA molecules to be stretched uniformly with a stretching factor close to 2 kb/µm (Michalet et al., 1997). For each motif, the following values were determined: the number of measured images (n), the theoretical calculated length (in kb), the mean measured length (kb), the standard deviation (sd, in kb), the coefficient of variation (CV, in %), the difference between measured and calculated length (delta, in kb).

### 2. Results

### Design of the high-resolution BRCA1 Genomic Morse Code v4.0

An electronic reconstruction of the designed BRCA1 GMC v4.0 is shown in Figure 1. The BRCA1 GMC covers a region of 200 kb, including the upstream genes NBR1, NBR2, LOC100133166, and TMEM106A, as well as the pseudogene BRCA1P1. The complete BRCA1 GMC is composed of 14 signals, and to facilitate GMC recognition and measurement, signals on the BRCA1+ NBR2 genes were grouped together in 8 specific patterns called "motifs" (m1b1 - m8b1).

### Characterization by Molecular Combing of a novel tandem repeat triplication of exons 1a, 1b and 2 in BRCA1

The presence of a large rearrangement on BRCA1 was first identified by visual inspection of the hybridization signals. A fraction of the detected signals showed a hybridization pattern differing from the normal pattern by the presence, between the S7 and S8 probe signals, of two additional pairs of signals corresponding to the color of the Synt1 and S7 probes (Figure 2A).

The signals were shown to arise from these probes by color swapping experiments, where the colors of some probes in the GMC are modified so as to observe the corresponding change in the hybridization signals. In one experiment, for example, the S7 probe was changed from green to blue and this resulted in the same change of color of the duplicated signal (Figure 2B).

The duplicated signal for the S7 probe was found to correspond to the full length of the S7 probe, while the additional signals for the Synt1 probe were found to correspond to only part of the Synt1 probe. This indicated the presence of a mutated allele, carrying an amplification of a region extending from the Synt1 probe to the gap between the S7 and S8 probes, along with an unmodified, wild-type allele in this sample.

Measurements were performed independently on signals from both alleles, the signals being attributed to either allele by the operator based on the hybridization pattern.

In one experiment, the SF was established from measurements of unmodified motifs (either from the wild-type allele or from unmodified regions in the mutated allele) to be 1.8 kb/µm. In the mutated allele, the distance from Synt1 to S8 was measured to be 38.5 kb, 14.9 kb longer than the expected size of 23.6 kb for a wild-type allele. This is expected to correspond to the measurement of the two extra copies of the amplified sequence, and the amplified sequence was thus determined to measure 7.4 kb. The 95 % confidence interval for the size, calculated as 7.4 kb +/- 2.sd√n (where n is the number of measurements used in the calculation), was found to be 6.6 kb - 8.2 kb.

In another experiment, the size of the first and second additional pairs of signals corresponding to Synt1 and S7 were measured to be 6.6 kb and 7.0 kb, respectively (from one end of the additional Synt1 probe signal to the other end of the proximal S7 probe signal) and the size of the region spanning both pairs of additional signals was measured to be 14.2 kb (from one end of the first additional Synt1 probe signal to the other end of the second additional S7 probe). Measuring the pairs of signals possibly excludes part of the amplified sequence (the part comprised between the S7 probe and the S8 probe) and was therefore considered an underestimate of the amplified sequence. The difference between the sum of both pairs measured individually and the direct measurement of the region spanning both pairs is a measurement of the part of the amplified sequence comprised between the S7 and S8 probes. This was measured to be 0.64 kb on average with a 95 % confidence interval, calculated as above, of 0.2 kb - 1.1 kb.

The 95 % confidence interval as above for the size of the region spanning both pairs measured directly, defined as above, is 13.4 kb - 14.9 kb. This measurement corresponds to two copies of the amplified sequence, with the exclusion of one copy of the part of the amplified sequence comprised between the S7 and S8 probes. The 95 % confidence interval for the size of the amplified sequence, when accounting for the part excluded from measurements using the determination above, is therefore 6.8 kb - 8.0 kb.

Here, we report the identification and characterization of a triplication of a 6 kb - 8.0 kb sequence, extending from intron 2 of the BRCA1 gene to the NBR2 gene. One extremity of the amplified sequence is within 2 kb of the extremity of the S7 probe (thus within genomic coordinates 41,278,700-41,280,700 in build hg19), while the other, as determined from the size of the amplified sequence is within genomic coordinates 41,270,700-41,274,700 in build hg19. This is the first report of a genomic amplification in this Alu-rich 5'-region of BRCA1, and the mutation is the second triplication reported so far in BRCA1 (Horgervost Cancer Research 2003, Sluiter Breast Cancer Research 2011).

### 3. Breakpoint prediction

As rearrangements may occur due to sequence homologies (van Binsbergen et al., 2012), we sought whether such homologies existed that may have contributed to the triplication, so as to more precisely define the potential location for the breakpoint. The sequences expected to contain the breakpoint as defined above by their genomic coordinates were submitted to local alignment search. The Lalign program was used (http://www.ch.embnet.org/software/LALIGN_form.html; implementing the algorithm of Huang and Miller, published in Adv. Appl. Math. (1991) 12:337-357). We used the blosum50 matrix, with an opening gap penalty of -30 and an extending gap penalty of -4. We assumed gaps were likely to strongly diminish interactions between sequences and so used a relatively high opening gap penalty. We set as criteria for homologies potentially involved in the breakpoint a minimum length of 200 bp with more than 80 % homology and containing a perfectly homologous stretch of at least 25 bp (not constituted of a poly-N segment where N is a given base). This search revealed one potential sequence homology, with 86.5 % over 296 bp, between the genome regions with genomic coordinates (in hg19): 41272510-41272805 and 41279769-41280064. These regions share a common 48 bp sequence (at positions 41279942-41279989 and 41272683-41272730). The size of the sequence between the two identical 48 bp sequences, 7.3 kb is perfectly compatible with the estimation of the amplified sequence.

### 4. Breakpoint characterization of the BRCA1 triplication by PCR and sequencing

Based on our estimation of the location of the breakpoint, we designed PCR primer pairs in order to specifically amplify the sequence containing the breakpoint in the sample with the triplication.

### PCR amplification

PCR and were performed in 50 µL reactions. Cycling conditions were chosen according to the polymerase and the length of the sequence to amplify. The Taq polymerase Expand High Fidelity from Roche was employed using following PCR conditions for each reaction: 200µM dNTP, 300µM primers, 1,5 mM MgCl2, 2.6U Taq. PCR amplification conditions were for the primer pairs F7/R7 and F9/R8: 10 cycles of (94°C for 15s, 57°C for 30s, 72°C for 2min), 30 cycles of (94°C for 15s, 57°C for 30s, 72°C for 2min), 72°C for 7min; for the other primer pairs: 95°C for 5min, 30 cycles of (94°C for 30s, 60°C for 60s, 72°C for 1min), 72°C for 10min. PCR products were analyzed on a 1% agarose gel containing SYBRsafe (InVitrogen) with 1 µg of the Marker Hyperladder I (Promega).

Primers have been designed with the Primer3 v.0.4.0 software (http://frodo.wi.mit.edu/primer3/) and synthesized by MWG/Eurogentec. Primer sequences and temperature of annealing are the following:
F7 5'- AGGGTTTCATCACGTTGGTC-3' 58°C, (SEQ ID NO:**3**)
R7 5'- GCAAATGTAGTGGGGACTTG -3' 57°C, (SEQ ID NO:**4**)
F9 5'- CTGCGCCTGGCTTAAGAT -3' 57°C, (SEQ ID NO:**5**)
R8 5'- GATGTGGGTGGGGTCAGA-3' 58°C, (SEQ ID NO:**6**)
F1 5'- ATAGGGTTTCATCACGTTGGTC-3' 60°C, (SEQ ID NO:**7**)
R1 5'- CTAATCTGGTGGGCACTTGG-3' 60°C, (SEQ ID NO:**8**)
F2 5'- GTCTTGAAGTCCTGATCTCGTG-3' 59°C, (SEQ ID NO:**9**)
R2 5'- GTGTCTAGCTTGGGGTTTGG-3' 60°C, (SEQ ID NO:**10**)
F3 5'- GAGATAGGGTTTCATCACGTTG-3' 59°C, (SEQ ID NO:**11**)
R3 5'- CAGATGGGGACTTGGAAAAC-3' 59°C, (SEQ ID NO:**12**)
F4 5'- GTTTCATCACGTTGGTCAGG-3' 59°C, (SEQ ID NO:**13**)
R4 5'- CTGAGTCAGATGGGGACTTG-3' 58°C, (SEQ ID NO:**14**)
F5 5'- GTTCAAGTTCAAGCGCTTCTC -3' 59°C, (SEQ ID NO:**15**)
F6 5'- CTGCCAGGTTCAAGTTCAAG -3' 58°C. (SEQ ID NO:**16**)
Following primers pairs were tested and validated by PCR: F7/R7 (SEQ ID No3./ SEQ ID No.4),
F9/R8 (SEQ ID No.5/ SEQ ID No.6), F1/R1 (SEQ ID No.7/ SEQ ID No.8), F1/R2 (SEQ ID
No.7/ SEQ ID No.10), F1/R3 (SEQ ID No.7/ SEQ ID No.12), F2/R7 (SEQ ID No.9/ SEQ ID
No.4), F3/R4 (SEQ ID No.11/ SEQ ID No.14), F3/R7 (SEQ ID No.11/ SEQ ID No.4), F4/R7
(SEQ ID No.13/ SEQ ID No.4), F5/R2 (SEQ ID No.15/ SEQ ID No.10), F5/R3 (SEQ ID No.15/
SEQ ID No.12), F6/R3 (SEQ ID No.16/ SEQ ID No.12), F7/R1 (SEQ ID No.3/ SEQ ID No.8), and F7/R2 (SEQ ID No.9/ SEQ ID No.10) F7/R3 (SEQ ID No.3/ SEQ ID No.12).

### DNA gel purification and sequencing

PCR amplified DNA fragments were purified with the QIAquick kit (QIAGEN), according to manufacturer's instructions. Purified fragments were then sequenced by Sanger sequencing (Plate-forme de séquençage et génomique, Institut Cochin Paris). DNA sequences were then analysed with the biological sequence alignment editor BioEdit (http://www.mbio.ncsu.edu/bioedit/bioedit.html) and bioinformatics analysis was performed with the software BLAST (http://blast.ncbi.nlnxmh.gov/Blast.cgi).

### Results

We were able to successfully amplify two DNA fragments by PCR, specific for the cell-line 10799001 bearing the BRCA1 triplication, but not for the control cell-line 40, employing primer pairs F7/R7 and F9/R8 (Figures 3B - 3C). An apparent 600bp DNA fragment was amplified by PCR with the primers F7/R7, and an apparent 400pb DNA fragment with primers F9/R8. Sequencing of the fragments resulted in 574 (primer F7), 561 (primer R7), 337 (primer F9) and 306 (primer R8) bases long DNA fragments. Bioinformatics analysis confirmed that the DNA fragment amplified by primers F7/R7 and F9/R8 was identical, with the F9/R8 being shorter than the F7/R7 fragment.

Sequence comparison with the reference human genome sequence showed that the amplified fragments were constituted by the region of intron 2 of BRCA1 extending from position 41,272,683towards the telomere and the 5' region of NBR2 extending from position 41,279,989towards the centromere connected by a 48 bp sequence common to both positions 41,272,683 in intron 2 of BRCA1 and 41,279,942, in the 5' region of NBR2 (figure 4). The 48bp common sequence is as follows: GAGGCAGGAGAATGGCGTGAACCCGGGAGGCGGAGCTTGCAGTGAGCC. (SEQ ID NO:**21**)

This is perfectly compatible with the triplication of the 7.3 kb-sequence fragment comprised between these two positions, with the breakpoint occurring in a stretch of perfect sequence identity. The exact physical mapping of the BRCA1 triplication is shown in figures. This is also consistent with the breakpoint prediction we established by sequence comparison based on the estimation of the breakpoint position.

### Direct PCR testing

Since PCR amplification using primer pairs F7/R7 and F9/R8 resulted in the amplification of PCR products in a control sample without amplification, we designed additional primer pairs that amplify products specifically in the sample bearing the amplification reported here.

As shown in Figure 5A, fragments specific for the BRCA1 triplication were obtained out of 8 primer pairs (F1/R1, F1/R2, F1/R3, F2/R7, F5/R2, F5/R3, F6/R3, F7/R2), with sizes consistent with the relative location of the primers and breakpoints. Primer pairs F5/R2, F5/R3 and F6/R3 showed amplification products only in the mutation positive cell-line 10799001, but not in the control cell-line 38.

Additional samples, from three unrelated patients (coming from different French regions, also unrelated to the patient from whom cell line 10799001 was established) where an amplification had been suspected following aCGH testing, were submitted to PCR amplification with primer pairs F5/R2, F5/R3 and F6/R3. A specific PCR product was observed, with the expected size for the amplification reported here, which was not observed in control samples (fig. 5B). These PCR products were sequenced and results were identical to cell-line 10799001. This confirmed the identical nature of the amplification, with the same breakpoint position, in four unrelated samples.

The primer pairs described here are examples of primer pairs that enable the specific detection of the reported breakpoint. Indeed, in a wild-type sample, the relative orientation of the forward and reverse primers of any of these pairs is such that no specific amplification is possible: the forward primer allows priming for a polymerization towards the centromere, while it is located upstream of the reverse primer. The tandem amplification brings an additional copy of the sequence corresponding to the forward primer (see Fig. 3). This additional copy being downstream of the reverse primer, the amplification of the sequence stretch between both primers becomes possible. Using such an approach, the man skilled in the art may design other primer pairs with equivalent properties. Such primer pairs must be constituted of
- one forward primer (oriented from telomere to centromere) located preferentially less than 5 kb, more preferentially less than 2 kb, even more preferentially less than 1 kb and even more preferentially less than 500 bp from the breakpoint location in the BRCA1 gene (i.e. between genomic positions 41,279,990 and 41,284,990; preferentially between positions 41,279,990 and 41,281,990; more preferentially between positions 41,279,990 and 41,280,990 and even more preferentially between positions 41,279,990 and 41,280,490); and
- one reverse primer (oriented from centromere to telomere)) located preferentially less than 5 kb, more preferentially less than 2 kb, even more preferentially less than 1 kb and even more preferentially less than 500 bp from the breakpoint location in the NBR2 gene (i.e. between genomic positions 41,267,683 and 41,272,683; preferentially between positions 41,270,683 and 41,272,683; more preferentially between positions 41,271,683 and 41,272,683 and even more preferentially between positions 41,272,183 and 41,272,683); where
- the forward primer is located upstream of the reverse primer.

### Discussion

The amplification reported here is the first report of a sequence amplification in the region of BRCA1 comprising exons 1a, 1b and 2 and the intervening introns, and the second triplication reported in the BRCA1 locus. Of note, this region of BRCA1 is very rich in repetitive sequences. The prior art relied on methods which have low detection capacity for such amplifications, either because they fail to cover regions rich in repetitive sequences, or because they fail to distinguish the copy number change induced by a triplication from that induced by a duplication.

Here, we show that using Molecular Combing or related direct mapping methods in such regions, it is possible to correctly detect and characterize such amplifications. The probe sets illustrated here are examples of probe sets which can be used for this purpose when using Molecular Combing. Adaptations of this design are possible and readily achievable by the man skilled in the art, whether for Molecular Combing or for related direct mapping methods. Using such methods, the amplification is typically detected either by a change in the succession of detected sequences or by an increase in length of the region of interest.

We also show that although in some regions such as the one involved in the triplication reported here, the presence of repetitive sequences makes specific PCR amplification challenging, with sufficient knowledge of the breakpoint location it is possible to obtain a product specific for the amplification. The nature of the product may be confirmed by sequencing, which unambiguously allows characterizing the resulting rearrangement.

The sufficient knowledge of the breakpoint location needed here may be obtained by careful analysis of mapping results obtained through Molecular Combing or related direct mapping methods. This may be further detailed by combining the mapping results with bioinformatics analysis to reveal potential breakpoint location. As described above, such potential breakpoint locations may be identified as sequences in the region determined to contain the breakpoint which show e.g. more than 80 % homology over more than 200 bp and contain an identical sequence stretch (non poly-N) of more than 25 bp.

In the case of the amplification of the region extending from intron 2 of BRCA1 to the 5' portion of NBR2 reported here, which appears to be a recurrent event, the amplification may be immediately characterized by using previously validated primer pairs, such as the ones we disclose here. Besides, the precise description of the breakpoint disclosed here would allow a man skilled in the art to use an alternative method (or PCR using different primer pairs) for the detection of this amplification.
In cases where such amplifications are reported to be recurrent, i.e. to occur in unrelated samples, systematic screening may also be considered. Direct testing for these recurrent amplifications without prior mapping is likely to efficiently reveal such an amplification in a sample.

The following numbered paragraphs represent various embodiments of the invention:
1. A method for *in vitro* prediction of a breakpoint associated with rearrangement, in particular large rearrangement, in a nucleic acid of a biological sample comprising nucleic acid representative of chromosomal nucleic acid, in particular human chromosomal nucleic acid, comprising the steps of:
   - mapping the nucleic acid of the biological sample , particularly using Molecular Combing or related direct mapping methods;
   - determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
   - determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
   - within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
   - and when such portions exist, exhibiting such sequence identity, reporting that such portions are likely to comprise the breakpoint for sequence rearrangement.
2. A method for detection of a breakpoint associated with rearrangement, in particular large rearrangement, in a nucleic acid of a biological sample comprising nucleic acid representative of chromosomal nucleic acid, in particular human chromosomal nucleic acid, comprising the steps of:
   - mapping the nucleic acid of the biological sample , particularly using Molecular Combing or related direct mapping methods;
   - determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
   - determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
   - within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
   - when such portions exist, exhibiting such sequence identity, concluding that such portions are likely to comprise the breakpoint for sequence rearrangement;
   - confirming, through molecular testing, in particular through PCR amplification or functionally related method and / or sequencing, the location of the breakpoint.
3. A method according to paragraph 1 or 2 comprising determining the homology and the identity within the nucleic acid of the sample by local alignment search, in particular by successive alignment searches.
4. A method according to any of paragraphs 1 to 3 wherein the search for homology excludes determining homology for poly-N segments i.e. repeats of a given nucleotide (N), where such a nucleotide is repeated at least 5 times consecutively.
5. A method according to any of paragraphs 1 to 4, wherein the level of homology is within the range of 85 to 95% of identical nucleotides.
6. A method according to any of paragraphs 1 to 5, where the homology is determined on a sequence having 200 to 500bp, in particular 200 to 300bp, in particular about 300 bp.
7. A method according to any of paragraphs 1 to 6, where the prediction or the detection of a breakpoint is associated with a rearrangement consisting of amplification of a nucleic acid sequence, deletion of a sequence in the genomic nucleic acid.
8. A method according to any of paragraphs 1 to 7, where the prediction or the detection of a breakpoint is performed after detection of a rearrangement in a nucleic acid sequence representative of a human genomic sequence.
9. A method according to any of paragraphs 1 to 8, where the prediction or the detection of a breakpoint is made on a locus of the genome which comprises a gene which is known to be associated with a disease or with a predisposition for a disease, such as genes associated with predisposition to breast and / or ovarian cancer, particularly BRCA1 and BRCA2, genes associated with Lynch syndrome or predisposition to colorectal cancer, particularly MSH2, MLH1, MSH6 and PMS2.
10. A method according to any of paragraphs 1 to 9, wherein the breakpoint is detected in the BRCA1 locus.
11. A method according to any of paragraphs 2 to 10, wherein the confirmation of the breakpoint is performed by PCR using primer pairs selected as follows:
   - one forward primer located preferentially less than 5 kb, more preferentially less than 2 kb, even more preferentially less than 1 kb and even more preferentially less than 500 bp from the location of the likely breakpoint at one end of the rearrangement and
   - one reverse primer located preferentially less than 5 kb, more preferentially less than 2 kb, even more preferentially less than 1 kb and even more preferentially less than 500 bp from the location of the likely breakpoint at the other end of the rearrangement and where the primers are oriented so that no amplification is possible by PCR in a wild-type sample.
12. A method for detecting a predisposition to a disease, or for the detection of a disease, in particular a cancer, especially a breast or ovarian cancer, which comprises performing the prediction or the detection of a breakpoint according to any of paragraphs 1 to 11.

### References

Casilli, F., Di Rocco, Z.C., Gad, S., Tournier, I., Stoppa-Lyonnet, D., Frebourg, T., and Tosi, M. (2002). Rapid detection of novel BRCA1 rearrangements in high-risk breast-ovarian cancer families using multiplex PCR of short fluorescent fragments. Hum Mutat 20, 218-226.
Dimalanta ET, Lim A, Runnheim R, Lamers C, Churas C, Forrest DK, de Pablo JJ, Graham MD, Coppersmith SN, Goldstein S, Schwartz DC (2004). "A 75 microfluidic system for large DNA molecule arrays." Anal Chem. 2004 Sep 15;76(18):5293-301.
Florijn RJ, Bonden LA, Vrolijk H, Wiegant J, Vaandrager JW, Baas F, den Dunnen JT, Tanke HJ, van Ommen GJ, Raap AK (1995). "High-resolution DNA Fiber-FISH for genomic DNA mapping and colour bar-coding of large genes." Hum Mol Genet. 1995 May;4(5):831-6.
Fransz PF, Alonso-Blanco C, Liharska TB, Peeters AJ, Zabel P, de Jong JH (1996). "High-resolution physical mapping in Arabidopsis thaliana and tomato by fluorescence in situ hybridization to extended DNA fibres." Plant J. 1996 Mar;9(3):421-30.
Gad, S., Aurias, A., Puget, N., Mairal, A., Schurra, C., Montagna, M., Pages, S., Caux, V., Mazoyer, S., Bensimon, A., et al. (2001). Color bar coding the BRCA1 gene on combed DNA: a useful strategy for detecting large gene rearrangements. Genes Chromosomes Cancer 31, 75-84.
Gad, S., Bieche, I., Barrois, M., Casilli, F., Pages-Berhouet, S., Dehainault, C., Gauthier-Villars, M., Bensimon, A., Aurias, A., Lidereau, R., et al. (2003). Characterization of a 161 kb deletion extending from the NBR1 to the BRCA1 genes in a French breast-ovarian cancer family. Hum Mutat 21, 654.
Gad, S., Caux-Moncoutier, V., Pages-Berhouet, S., Gauthier-Villars, M., Coupier, I., Pujol, P., Frenay, M., Gilbert, B., Maugard, C., Bignon, Y.J., et al. (2002a). Significant contribution of large BRCA1 gene rearrangements in 120 French breast and ovarian cancer families. Oncogene 21, 6841-6847.
Gad, S., Klinger, M., Caux-Moncoutier, V., Pages-Berhouet, S., Gauthier-Villars, M., Coupier, I., Bensimon, A., Aurias, A., and Stoppa-Lyonnet, D. (2002b). Bar code screening on combed DNA for large rearrangements of the BRCA1 and BRCA2 genes in French breast cancer families. J Med Genet39, 817-821.
Gill P, Ghaemi A. Nucleic acid isothermal amplification technologies: a review. Nucleosides Nucleotides Nucleic Acids. 2008 Mar;27(3):224-43.
Haaf T, Ward DC (1994)."Structural analysis of alpha-satellite DNA and centromere proteins using extended chromatin and chromosomes." Hum Mol Genet. 1994 May;3(5):697-709.
Heiskanen M, Kallioniemi O, Palotie A (1996). "Fiber-FISH: experiences and a refined protocol." Genet Anal. 1996 Mar;12(5-6):179-84.
Heiskanen M, Karhu R, Hellsten E, Peltonen L, Kallioniemi OP, Palotie A (1994). "High resolution mapping using fluorescence in situ hybridization to extended DNA fibers prepared from agarose-embedded cells." Biotechniques. 1994 Nov; 17(5):928-9, 932-3.
Heng HH, Squire J, Tsui LC (1992). "High-resolution mapping of mammalian 30 genes by in situ hybridization to free chromatin." Proc Natl Acad Sci U S A. 1992 Oct 15;89(20):9509-13.
Herrick, J., and Bensimon, A. (2009). Introduction to molecular combing: genomics, DNA replication, and cancer. Methods Mol Biol 521, 71-101.
Hofmann, W., Wappenschmidt, B., Berhane, S., Schmutzler, R., and Scherneck, S. (2002). Detection of large rearrangements of exons 13 and 22 in the BRCA1 gene in German families. J Med Genet 39, E36.
Hogervorst FB, Nederlof PM, Gille JJ, McElgunn CJ, Grippeling M, Pruntel R, Regnerus R, van Welsem T, van Spaendonk R, Menko FH, Kluijt I, Dommering C, Verhoef S, Schouten JP, van't Veer LJ, Pals G (2003). Large genomic deletions and duplications in the BRCA1 gene identified by a novel quantitative method. Cancer Res. 2003 Apr 1;63(7):1449-53.
Jing J, Reed J, Huang J, Hu X, Clarke V, Edington J, Housman D, Anantharaman TS, Huff EJ, Mishra B, Porter B, Shenker A, Wolfson E, Hiort C, Kantor R, Aston C, Schwartz DC (1998). "Automated high resolution optical mapping using arrayed, fluid-fixed DNA molecules." Proc Natl Acad Sci U S A. 1998 Jul 7;95(14):8046-51.
King, M.C., Marks, J.H., and Mandell, J.B. (2003). Breast and ovarian cancer risks due to inherited mutations in BRCA1 and BRCA2. Science 302, 643-646.
Larson JW, Yantz GR, Zhong Q, Charnas R, D'Antoni CM, Gallo MV, Gillis KA, Neely LA, Phillips KM, Wong GG, Gullans SR, Gilmanshin R (2006). "Single DNA molecule stretching in sudden mixed shear and elongational microflows." Lab Chip. 2006 Sep;6(9):1187-99. Epub 2006 Jul 7.
Mann SM, Burkin DJ, Grin DK, Ferguson-Smith MA (1997). "A fast, novel approach for DNA fibre-fluorescence in situ hybridization analysis." Chromosome Res. 1997 Apr;5(2): 145-7.
Mazoyer, S. (2005). Genomic rearrangements in the BRCA1 and BRCA2 genes. Hum Mutat 25, 415-422.
Michalet X, Ekong R, Fougerousse F, Rousseaux S, Schurra C, Hornigold N, van Slegtenhorst M, Wolfe J, Povey S, Beckmann JS, Bensimon A (1997). "Dynamic molecular combing: stretching the whole human genome for highresolution studies." Science.; 277(5331):1518-23.
Nathanson, K.L., Wooster, R., and Weber, B.L. (2001). Breast cancer genetics: what we know and what we need. Nat Med 7, 552-556.
Palotie A, Heiskanen M, Laan M, Horelli-Kuitunen N (1996). "High-resolution fluorescence in situ hybridization: a new approach in genome mapping." Ann Med. 1996 Apr;28(2): 101-6. 77 Parra I, Windle B (1993). "High resolution visual mapping of stretched DNA by fluorescent hybridization." Nat Genet. 1993 Sep;5(1): 17-21.
Raap AK (1998). "Advances in fluorescence in situ hybridization." Mutat Res. 1998 May 25;400(1-2):287 -98.
Rouleau, E., Lefol, C., Tozlu, S., Andrieu, C., Guy, C., Copigny, F., Nogues, C., Bieche, I., and Lidereau, R. (2007). High-resolution oligonucleotide array-CGH applied to the detection and characterization of large rearrangements in the hereditary breast cancer gene BRCA1. Clin Genet 72,199-207.
Samad A, Huff EF, Cai W, Schwartz DC (1995). "Optical mapping: a novel, single-molecule approach to genomic analysis." Genome Res. 1995 Aug;5(1):1-4.
Schouten JP, McElgunn CJ, Waaijer R, Zwijnenburg D, Diepvens F, Pals G. Relative quantification of 40 nucleic acid sequences by multiplex ligation-dependent probe amplification. Nucleic Acids Res. 2002 Jun 15;30(12):e57
Schurra, C., and Bensimon, A. (2009). Combing genomic DNA for structural and functional studies. Methods Mol Biol 464, 71-90.
Schwartz DC, Li X, Hernandez LI, Ramnarain SP, Huff EJ, Wang YK (1996). "Ordered restriction maps of Saccharomyces cerevisiae chromosomes constructed by optical mapping." Science. 1993 Oct 1;262(5130):110-4.
Sluiter MD, van Rensburg EJ (2011). Large genomic rearrangements of the BRCA1 andBRCA2 genes: review of the literature and report of a novel BRCA1 mutation. Breast Cancer Res Treat. 2011 Jan; 125(2):325-49. doi: 10.1007/s10549-010-0817-z. Epub 2010 Mar 16.
Staaf, J., Torngren, T., Rambech, E., Johansson, U., Persson, C., Sellberg, G., Tellhed, L., Nilbert, M., and Borg, A. (2008). Detection and precise mapping of germline rearrangements in BRCA1, BRCA2,MSH2, and MLH1 using zoom-in array comparative genomic hybridization (aCGH). Hum Mutat 29,555-564.
Szabo, C., Masiello, A., Ryan, J.F., and Brody, L.C. (2000). The breast cancer information core:database design, structure, and scope. Hum Mutat 16, 123-131.
Vaandrager JW, Schuuring E, Kluin-Nelemans HC, Dyer MJ, Raap AK, Kluin PM (1996). "DNA fiber fluorescence in situ hybridization analysis of immunoglobulin class switching in B-cell neoplasia: aberrant CH gene rearrangements in follicle center-cell lymphoma." Blood. 1998 Oct 15;92(8):2871- 8.
van Binsbergen E. Origins and breakpoint analyses of copy number variations: up close and personal. Cytogenet Genome Res. 2011; 135(3-4):271-6. doi:10.1159/000330267. Epub 2011 Aug 12.
Walsh, T., Lee, M.K., Casadei, S., Thornton, A.M., Stray, S.M., Pennil, C., Nord, A.S., Mandell, J.B., Swisher, E.M., and King, M.C. (2010). Detection of inherited mutations for breast and ovarian cancer using genomic capture and massively parallel sequencing. Proc Natl Acad Sci U S A 107, 12629-12633.
Wiegant J, Kalle W, Mullenders L, Brookes S, Hoovers JM, Dauwerse JG, van Ommen GJ, Raap AK (1996). "High-resolution in situ hybridization using DNA halo preparations." Hum Mol Genet. 1992 Nov;1(8):587-91.
Murphy PD, Allen AC, Alvares CP, Critz BS, Olson SJ, Schelter DB, Zeng B: Coding sequences of the human BRCA1 gene US 5,750,400 Skolnick MH, Goldgar DE, Miki Y, Swenson J, Kamb A, Harshman KD, Shattuck-eidens DM, Tavtigian SV, Wiseman RW, Futreal AP: 17q-linked breast and ovarian cancer susceptibility gene US 5,710,001

## Claims

1. A method for *in vitro* prediction of a breakpoint associated with rearrangement, in particular large rearrangement, in a nucleic acid of a biological sample comprising nucleic acid representative of chromosomal nucleic acid, in particular human chromosomal nucleic acid, comprising the steps of:
- mapping the nucleic acid of the biological sample , particularly using Molecular Combing or related direct mapping methods;
- determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
- determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
- within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
- and when such portions exist, exhibiting such sequence identity, reporting that such portions are likely to comprise the breakpoint for sequence rearrangement.

2. A method for detection of a breakpoint associated with rearrangement, in particular large rearrangement, in a nucleic acid of a biological sample comprising nucleic acid representative of chromosomal nucleic acid, in particular human chromosomal nucleic acid, comprising the steps of:
- mapping the nucleic acid of the biological sample , particularly using Molecular Combing or related direct mapping methods;
- determining the size and/or confidence interval for the size of the rearrangement, the location and/or confidence interval for the location of one breakpoint at one end of the rearrangement, and the location and/or confidence intervals for the location of the breakpoint at the other end of the rearranged sequence;
- determining sequence homology between the predicted sequences of the locations determined for the breakpoints, such predicted sequences being taken from reference databases, in particular in the human reference genome, by determining presence of homologous sequence stretches with nucleotide identity of 80 to 98% of the nucleotides over the length of the sequence stretch, when each sequence stretch for which homology is determined in the nucleic acid has a length of at least 200 bp;
- within said identified homologous sequence stretches, determining strict sequence identity over a portion of the homologous nucleic acid sequences, said strict identity existing over a sequence portion of about 25bp to about 80bp, in particular over a sequence of at least 30 or at least 40 or at least 45 bp, and especially less than 80pb;
- when such portions exist, exhibiting such sequence identity, concluding that such portions are likely to comprise the breakpoint for sequence rearrangement;
- confirming, through molecular testing, in particular through PCR amplification or functionally related method and / or sequencing, the location of the breakpoint.

3. A method according to claim 1 or 2 comprising determining the homology and the identity within the nucleic acid of the sample by local alignment search, in particular by successive alignment searches.

4. A method according to any of claims 1 to 3 wherein the search for homology excludes determining homology for poly-N segments i.e. repeats of a given nucleotide (N), where such a nucleotide is repeated at least 5 times consecutively.

5. A method according to any of claims 1 to 4, wherein the level of homology is within the range of 85 to 95% of identical nucleotides.

6. A method according to any of claims 1 to 5, where the homology is determined on a sequence having 200 to 500bp, in particular 200 to 300bp, in particular about 300 bp.

7. A method according to any of claims 1 to 6, where the prediction or the detection of a breakpoint is associated with a rearrangement consisting of amplification of a nucleic acid sequence, deletion of a sequence in the genomic nucleic acid.

8. A method according to any of claims 1 to 7, where the prediction or the detection of a breakpoint is performed after detection of a rearrangement in a nucleic acid sequence representative of a human genomic sequence.

9. A method according to any of claims 1 to 8, where the prediction or the detection of a breakpoint is made on a locus of the genome which comprises a gene which is known to be associated with a disease or with a predisposition for a disease, such as genes associated with predisposition to breast and / or ovarian cancer, particularly BRCA1 and BRCA2, genes associated with Lynch syndrome or predisposition to colorectal cancer, particularly MSH2, MLH1, MSH6 and PMS2.

10. A method according to any of claims 1 to 9, wherein the breakpoint is detected in the BRCA1 locus.

11. A method according to any of claims 2 to 10, wherein the confirmation of the breakpoint is performed by PCR using primer pairs selected as follows:
- one forward primer located preferentially less than 5 kb, more preferentially less than 2 kb, even more preferentially less than 1 kb and even more preferentially less than 500 bp from the location of the likely breakpoint at one end of the rearrangement and
- one reverse primer located preferentially less than 5 kb, more preferentially less than 2 kb, even more preferentially less than 1 kb and even more preferentially less than 500 bp from the location of the likely breakpoint at the other end of the rearrangement and where the primers are oriented so that no amplification is possible by PCR in a wild-type sample.

12. A method for detecting a predisposition to a disease, or for the detection of a disease, in particular a cancer, especially a breast or ovarian cancer, which comprises performing the prediction or the detection of a breakpoint according to any of claims 1 to 11.

## Patentansprüche

1. Verfahren zur *in vitro* Vorhersage eines Bruchpunkts in Zusammenhang mit einer Umlagerung, insbesondere großen Umlagerung, in einer Nukleinsäure einer biologischen Probe, die für chromosomale Nukleinsäure, insbesondere humane chromosomale Nukleinsäure, repräsentative Nukleinsäure umfasst, umfassend die Schritte:
- Kartieren der Nukleinsäure der biologischen Probe, insbesondere unter Verwendung von Molecular Combing oder ähnlichen Verfahren zur Direktkartierung,
- Bestimmen der Größe und/oder des Konfidenzintervalls für die Größe der Umlagerung, der Lage und/oder des Konfidenzintervalls für die Lage eines Bruchpunkts an einem Ende der Umlagerung und der Lage und/oder des Konfidenzintervalls für die Lage des Bruchpunkts am anderen Ende der umgelagerten Sequenz,
- Bestimmen einer Sequenzhomologie zwischen den vorhergesagten Sequenzen der für die Bruchpunkte bestimmten Lagen, wobei solche vorhergesagten Sequenzen aus Referenzdatenbanken, insbesondere im Referenzgenom des Menschen, herangezogen werden, durch Bestimmen eines Vorliegens von homologen Sequenzstrecken mit einer Nukleotididentität von 80 bis 98 % der Nukleotide über die Länge der Sequenzstrecke, wenn jede Sequenzstrecke, für die eine Homologie in der Nukleinsäure bestimmt wird, eine Länge von mindestens 200 bp aufweist,
- innerhalb der identifizierten homologen Sequenzstrecken, Bestimmen einer strikten Sequenzidentität über einen Abschnitt der homologen Nukleinsäuresequenzen, wobei die strikte Identität über einen Sequenzabschnitt von ungefähr 25 bp bis ungefähr 80 bp, insbesondere über eine Sequenz von mindestens 30 oder mindestens 40 oder mindestens 45 bp, und speziell weniger als 80 bp vorliegt,
- und wenn solche Abschnitte vorliegen, Aufzeigen einer solchen Sequenzidentität und Angeben, dass solche Abschnitte wahrscheinlich den Bruchpunkt für eine Sequenzumlagerung umfassen.

2. Verfahren zum Erfassen eines Bruchpunkts in Zusammenhang mit einer Umlagerung, insbesondere großen Umlagerung, in einer Nukleinsäure einer biologischen Probe, die für chromosomale Nukleinsäure, insbesondere humane chromosomale Nukleinsäure, repräsentative Nukleinsäure umfasst, umfassend die Schritte:
- Kartieren der Nukleinsäure der biologischen Probe, insbesondere unter Verwendung von Molecular Combing oder ähnlichen Verfahren zur Direktkartierung,
- Bestimmen der Größe und/oder des Konfidenzintervalls für die Größe der Umlagerung, der Lage und/oder des Konfidenzintervalls für die Lage eines Bruchpunkts an einem Ende der Umlagerung und der Lage und/oder des Konfidenzintervalls für die Lage des Bruchpunkts am anderen Ende der umgelagerten Sequenz,
- Bestimmen einer Sequenzhomologie zwischen den vorhergesagten Sequenzen der für die Bruchpunkte bestimmten Lagen, wobei solche vorhergesagten Sequenzen aus Referenzdatenbanken, insbesondere im Referenzgenom des Menschen, herangezogen werden, durch Bestimmen eines Vorliegens von homologen Sequenzstrecken mit einer Nukleotididentität von 80 bis 98 % der Nukleotide über die Länge der Sequenzstrecke, wenn jede Sequenzstrecke, für die eine Homologie in der Nukleinsäure bestimmt wird, eine Länge von mindestens 200 bp aufweist,
- innerhalb der identifizierten homologen Sequenzstrecken, Bestimmen einer strikten Sequenzidentität über einen Abschnitt der homologen Nukleinsäuresequenzen, wobei die strikte Identität über einen Sequenzabschnitt von ungefähr 25 bp bis ungefähr 80 bp, insbesondere über eine Sequenz von mindestens 30 oder mindestens 40 oder mindestens 45 bp, und speziell weniger als 80 bp vorliegt,
- wenn solche Abschnitte vorliegen, Aufzeigen einer solchen Sequenzidentität und Schlussfolgerung, dass solche Abschnitte wahrscheinlich den Bruchpunkt für eine Sequenzumlagerung umfassen,
- Bestätigen der Lage des Bruchpunkts durch Molekulartests, insbesondere durch PCR-Amplifizierung oder ein funktionell ähnliches Verfahren und/oder Sequenzierung.

3. Verfahren nach Anspruch 1 oder 2, umfassend Bestimmen der Homologie und der Identität in der Nukleinsäure der Probe durch lokale Alignmentuntersuchung, insbesondere sukzessive Alignmentuntersuchungen.

4. Verfahren nach einem der Ansprüche 1 bis 3, wobei die Untersuchung auf Homologie ein Bestimmen der Homologie für Poly-N-Segmente, d.h. Wiederholungen eines gegebenen Nukleotids (N), worin ein solches Nukleotid mindestens 5-mal nacheinander wiederholt ist, ausschließt.

5. Verfahren nach einem der Ansprüche 1 bis 4, wobei der Grad an Homologie in einem Bereich von 85 bis 95 % identischer Nukleotide liegt.

6. Verfahren nach einem der Ansprüche 1 bis 5, wobei die Homologie an einer Sequenz mit 200 bis 500 bp, insbesondere 200 bis 300 bp, insbesondere ungefähr 300 bp bestimmt wird.

7. Verfahren nach einem der Ansprüche 1 bis 6, wobei die Vorhersage oder die Erfassung eines Bruchpunkts mit einer Umlagerung in Zusammenhang steht, die in einer Amplifizierung einer Nukleinsäuresequenz, einer Deletion einer Sequenz in der genomischen Nukleinsäure besteht.

8. Verfahren nach einem der Ansprüche 1 bis 7, wobei die Vorhersage oder die Erfassung eines Bruchpunkts nach Erfassung einer Umlagerung in einer für eine humane Genomsequenz repräsentativen Nukleinsäuresequenz durchgeführt wird.

9. Verfahren nach einem der Ansprüche 1 bis 8, wobei die Vorhersage oder die Erfassung eines Bruchpunkts an einer Stelle des Genoms vorgenommen wird, die ein Gen umfasst, von dem bekannt ist, dass es mit einer Krankheit oder mit einer Prädisposition für eine Krankheit in Zusammenhang steht, wie Gene in Zusammenhang mit einer Prädisposition für Brustkrebs und/oder Eierstockkrebs, insbesondere BRCA1 und BRCA2, Gene in Zusammenhang mit einem Lynch-Syndrom oder einer Prädisposition für kolorektalen Krebs, insbesondere MSH2, MLH1, MSH6 und PMS2.

10. Verfahren nach einem der Ansprüche 1 bis 9, wobei der Bruchpunkt am BRCA1-Lokus erfasst wird.

11. Verfahren nach einem der Ansprüche 2 bis 10, wobei die Bestätigung des Bruchpunkts mit PCR durchgeführt wird, wobei Primer-Paare verwendet werden, die wie folgt ausgewählt sind:
- ein Vorwärtsprimer, der vorzugsweise weniger als 5 kb, bevorzugt weniger als 2 kb, besonders bevorzugt weniger als 1 kb und insbesondere bevorzugt weniger als 500 bp von der Lage des wahrscheinlichen Bruchpunkts an einem Ende der Umlagerung gelegen ist, und
- ein Rückwärtsprimer, der vorzugsweise weniger als 5 kb, bevorzugt weniger als 2 kb, besonders bevorzugt weniger als 1 kb und insbesondere bevorzugt weniger als 500 bp von der Lage des wahrscheinlichen Bruchpunkts am anderen Ende der Umlagerung gelegen ist, und wobei die Primer so orientiert sind, dass keine Amplifizierung durch PCR in einer Probe vom Wildtyp möglich ist.

12. Verfahren zum Erfassen einer Prädisposition für eine Krankheit oder zum Erfassen einer Krankheit, insbesondere eines Krebses, speziell von Brustkrebs oder Eierstockkrebs, das Durchführen der Vorhersage oder der Erfassung eines Bruchpunkts nach einem der Ansprüche 1 bis 11 umfasst.

## Revendications

1. Procédé de prédiction *in vitro* d'un point de cassure associé à un réarrangement, en particulier un grand réarrangement, dans un acide nucléique d'un échantillon biologique comprenant de l'acide nucléique représentatif d'acide nucléique chromosomique, en particulier d'acide nucléique chromosomique humain, comprenant les étapes de :
- cartographie de l'acide nucléique de l'échantillon biologique, en particulier en utilisant des procédés de peignage moléculaire ou de cartographie directe apparentée ;
- détermination de la taille et/ou de l'intervalle de confiance pour la taille du réarrangement, de l'emplacement et/ou de l'intervalle de confiance pour l'emplacement d'un point de cassure à une extrémité du réarrangement, et de l'emplacement et/ou de l'intervalle de confiance pour l'emplacement du point de cassure à l'autre extrémité de la séquence réarrangée ;
- détermination de l'homologie de séquence entre les séquences prédites des emplacements déterminés pour les points de cassure, de telles séquences prédites étant prises dans des bases de données de référence, en particulier dans le génome de référence humain, par détermination de la présence d'étendues de séquences homologues ayant une identité nucléotidique de 80 à 98 % des nucléotides sur la longueur de l'étendue de la séquence, lorsque chaque étendue de séquence pour laquelle l'homologie est déterminée dans l'acide nucléique a une longueur d'au moins 200 pb ;
- à l'intérieur desdites étendues de séquences homologues identifiées, la détermination d'une identité de séquence stricte sur une partie des séquences d'acide nucléique homologues, ladite identité stricte existant sur une partie de séquence d'environ 25 pb à environ 80 pb, en particulier sur une séquence d'au moins 30 ou d'au moins 40 ou d'au moins 45 pb, et notamment inférieure à 80 pb ;
- et quand de telles parties existent, présentant une telle identité de séquence, la signalisation que de telles parties sont susceptibles de comporter le point de cassure pour le réarrangement de séquence.

2. Procédé de détection d'un point de cassure associé à un réarrangement, en particulier un grand réarrangement, dans un acide nucléique d'un échantillon biologique comprenant de l'acide nucléique représentatif d'acide nucléique chromosomique, en particulier d'acide nucléique chromosomique humain, comprenant les étapes de :
- cartographie de l'acide nucléique de l'échantillon biologique, en particulier en utilisant des procédés de peignage moléculaire ou de cartographie directe apparentée ;
- détermination de la taille et/ou de l'intervalle de confiance pour la taille du réarrangement, de l'emplacement et/ou de l'intervalle de confiance pour l'emplacement d'un point de cassure à une extrémité du réarrangement, et de l'emplacement et/ou de l'intervalle de confiance pour l'emplacement du point de cassure à l'autre extrémité de la séquence réarrangée ;
- détermination de l'homologie de séquence entre les séquences prédites des emplacements déterminés pour les points de cassure, de telles séquences prédites étant prises dans des bases de données de référence, en particulier dans le génome de référence humain, par détermination de la présence d'étendues de séquences homologues ayant une identité nucléotidique de 80 à 98 % des nucléotides sur la longueur de l'étendue de la séquence, lorsque chaque étendue de séquence pour laquelle l'homologie est déterminée dans l'acide nucléique a une longueur d'au moins 200 pb ;
- à l'intérieur desdites étendues de séquences homologues identifiées, la détermination d'une identité de séquence stricte sur une partie des séquences d'acide nucléique homologues, ladite identité stricte existant sur une partie de séquence d'environ 25 pb à environ 80 pb, en particulier sur une séquence d'au moins 30 ou d'au moins 40 ou d'au moins 45 pb, et notamment inférieure à 80 pb ;
- quand de telles parties existent, présentant une telle identité de séquence, la conclusion que de telles parties sont susceptibles de comporter le point de cassure pour le réarrangement de séquence ;
- la confirmation, par test moléculaire, en particulier par une amplification PCR ou un procédé fonctionnellement apparenté et/ou un séquençage, de l'emplacement du point de cassure.

3. Procédé selon la revendication 1 ou 2, comprenant la détermination de l'homologie et de l'identité dans l'acide nucléique de l'échantillon par une recherche par alignement local, en particulier par des recherches par alignements successifs.

4. Procédé selon l'une quelconque des revendications 1 à 3, dans lequel la recherche d'homologie exclut la détermination de l'homologie pour les segments poly-N, c'est-à-dire les répétitions d'un nucléotide (N) donné, où un tel nucléotide est répété au moins 5 fois consécutivement.

5. Procédé selon l'une quelconque des revendications 1 à 4, dans lequel le niveau d'homologie est compris dans la plage de 85 à 95 % de nucléotides identiques.

6. Procédé selon l'une quelconque des revendications 1 à 5, où l'homologie est déterminée sur une séquence ayant 200 à 500 pb, en particulier 200 à 300 pb, en particulier environ 300 pb.

7. Procédé selon l'une quelconque des revendications 1 à 6, où la prédiction ou la détection d'un point de cassure est associée à un réarrangement constitué par une amplification d'une séquence d'acide nucléique, une délétion d'une séquence dans l'acide nucléique génomique.

8. Procédé selon l'une quelconque des revendications 1 à 7, où la prédiction ou la détection d'un point de cassure est effectuée après détection d'un réarrangement dans une séquence d'acide nucléique représentative d'une séquence génomique humaine.

9. Procédé selon l'une quelconque des revendications 1 à 8, où la prédiction ou la détection d'un point de cassure est réalisée sur un locus du génome qui comprend un gène qui est connu pour être associé à une maladie ou à une prédisposition à une maladie, tel que les gènes associés à une prédisposition au cancer du sein et/ou de l'ovaire, en particulier BRCA1 et BRCA2, les gènes associés au syndrome de Lynch ou à une prédisposition au cancer colorectal, en particulier MSH2, MLH1, MSH6 et PMS2.

10. Procédé selon l'une quelconque des revendications 1 à 9, dans lequel le point de cassure est détecté dans le locus BRCA1.

11. Procédé selon l'une quelconque des revendications 2 à 10, dans lequel la confirmation du point de cassure est effectuée par PCR à l'aide de paires d'amorces choisies comme suit :
- une amorce sens située préférentiellement à moins de 5 kb, plus préférentiellement à moins de 2 kb, encore plus préférentiellement à moins de 1 kb et encore plus préférentiellement à moins de 500 pb de l'emplacement du point de cassure probable à une extrémité du réarrangement, et
- une amorce inverse située préférentiellement à moins de 5 kb, plus préférentiellement à moins de 2 kb, encore plus préférentiellement à moins de 1 kb et encore plus préférentiellement à moins de 500 pb de l'emplacement du point de cassure probable à l'autre extrémité du réarrangement et où les amorces sont orientées de sorte qu'aucune amplification n'est possible par PCR dans un échantillon de type sauvage.

12. Procédé pour détecter une prédisposition à une maladie ou pour la détection d'une maladie, en particulier un cancer, en particulier un cancer du sein ou de l'ovaire, qui comprend la réalisation de la prédiction ou la détection d'un point de cassure selon l'une quelconque des revendications 1 à 11.
